# EUROPEAN PATENT APPLICATION

(11) **EP 4 129 268 A1**
(43) Date of publication of application: **08.02.2023**
(21) Application number: 21781987.9
(22) Date of filing: 25.03.2021
(51) Int. Cl.: A61K 8/891, A61K 8/31, A61K 8/34, A61K 8/81, A61Q 19/00, A61Q 19/08

(54) **SKIN TREATMENT METHOD**

(30) Priority: 01.04.2020 JP 2020066051
(71) Applicant: Kao Corporation, Chuo-ku Tokyo 103-8210 (JP)
(72) Inventor: FUKUDA, Teruyuki, Wakayama-shi, Wakayama 640-8580 (JP); IIDA, Masayuki, Odawara-shi, Kanagawa 250-0002 (JP); ITO, Motoaki, Odawara-shi, Kanagawa 250-0002 (JP)
(74) Representative: Hoffmann Eitle
(86) International application number: PCT/JP2021/012643
(87) International publication number: WO 2021/200574

(57) **Abstract**

The present invention relates to a skin treatment method including the step 1 of applying fine liquid droplets of a liquid composition containing a polymer A and a volatile solvent B to skin and the step 2 of drying the fine liquid droplets applied to the skin in the step 1 to contract the skin in association with the drying, in which a deformation ratio of the polymer A as measured by the method (I) is from 0.3 to 1, and a mass of the fine liquid droplets applied to the skin in the step 1 as measured per one droplet thereof is from 0.001 to 100 µg; and a makeup method using the skin treatment method.

## Description

### FIELD OF THE INVENTION

The present invention relates to a skin treatment method.

### BACKGROUND OF THE INVENTION

As aging phenomena of skin, there may be mentioned wrinkles that are present around eyes, around mouth, and in forehead, neck, etc., sagging of a face line, and the like. Such aging phenomena have been caused by a plurality of factors, such as the change in skin tissue, reduction of bone density, exposure to ultraviolet light, etc.

As the method of making the wrinkles or sagging inconspicuous, there is known cosmetic surgery. The cosmetic surgery has been conducted by an invasive surgical procedure, such as a face lift surgery, etc., or a low-invasive non-surgical procedure, such as injection treatment with a filler for removal of face wrinkles, etc. However, these procedures have been sometimes accompanied with difficulty in shape control, and have therefore posed problems, such as a large physical burden on practitioners.

In these circumstances, there have been proposed the methods for improving wrinkles by non-invasive procedures.

For example, JP 2005-200320A (Patent Literature 1) aims at providing a skin external preparation for wrinkle improvement which is excellent in wrinkle-improving effect, usability and a sense of use, and discloses a skin external preparation for wrinkle improvement which contains a polymer water dispersion prepared by dispersing a water-insoluble film-forming polymer in water, in which the film-forming polymer contains a polyurethane having a film shrinkage rate of not more than 20% and an acrylic polymer having a film shrinkage rate of not more than 20% as main components thereof.

JP 2006-169145A (Patent Literature 2) aims at providing a wrinkle smoothing agent that is capable of not only exerting a good wrinkle smoothing effect in a short time, but also imposing a less burden on the other skin portions, and a wrinkle smoothing method, and discloses a skin wrinkle smoothing agent that is formed of a liquid or paste-like composition containing a polymer, and a solvent or a dispersion medium, and further capable of exhibiting a force for smoothing wrinkles by shrinkage when volatilizing the solvent or dispersion medium therefrom after being applied to skin, and a wrinkle smoothing method in which the wrinkle smoothing agent is applied to a skin portion where the wrinkles are present, or surrounding portions thereof.

JP 2016-532482A (Patent Literature 3) aims at providing an applicator capable of applying a composition that can reduce occurrence of wrinkles on skin while balancing contraction of skin, film flexibility, contraction resiliency, etc., and provides an applicator for applying a skin smoothing composition, which includes a head and a body. In the Patent Literature 3, it is described that the head has a portion whose surface is coated with specific flock fibers, and the skin smoothing composition contains sodium silicate, polyvalent silicate and water, and also described that the flock fibers can be contacted with the skin smoothing composition, and then the skin smoothing composition can be applied in the form of a uniform film to the skin through the flock fibers.

### SUMMARY OF THE INVENTION

The present invention relates to a skin treatment method including:
Step 1: applying fine liquid droplets of a liquid composition containing a polymer A and a volatile solvent B to skin; and
Step 2: drying the fine liquid droplets applied to the skin in the step 1 to contract the skin in association with the drying, in which:
   a deformation ratio of the polymer A as measured by the following method (I) is not less than 0.3 and not more than 1; and
   a mass of the fine liquid droplets applied to the skin in the step 1 as measured per one droplet thereof is not less than 0.001 µg and not more than 100 µg,
   Method (I): dissolving the polymer A in the volatile solvent B to prepare a polymer solution having a concentration of 10% by mass; applying 0.005 g of the thus prepared polymer solution to a region of a surface of a polyethylene sheet having a width of 20 mm, a length of 100 mm and a thickness of 0.03 mm, the region having a width of 20 mm and a length of 50 mm which extends from an end of a short side of the polyethylene sheet along a length direction thereof and drying the polymer solution applied to the polyethylene sheet in a constant temperature oven set at 40 °C for 10 minutes to prepare the polyethylene sheet with a polymer film,
   in which when a length of a portion of the polyethylene sheet to which the polymer solution is applied is represented by L1, and a length of a portion of the polyethylene sheet with the polymer film which undergoes deformation by drying the polymer solution applied thereto is represented by L2, a ratio of L2 to L1 [L2/L1] is defined as the deformation ratio of the polymer A.

### BRIEF DESCRIPTION OF THE DRAWINGS

FIG. 1 is an explanatory view of a method of measuring a deformation ratio (L2/L1) of the polymer A by the method (I).
FIG. 2 is an explanatory view showing an example of a contraction occurrence mechanism of skin according to the present invention.
FIG. 3 is an explanatory view showing an example of a print pattern used for applying the fine liquid droplets in the step 1.
FIG. 4 is an explanatory view showing another example of the print pattern used for applying the fine liquid droplets in the step 1.
FIG. 5 is an explanatory view showing an even another example of the print pattern used for applying the fine liquid droplets in the step 1.
FIG. 6 is a cross-sectional view schematically showing the condition in which the fine liquid droplets are applied onto the skin by applying the fine liquid droplets in the step 1 using the print pattern shown in FIG. 4 or FIG. 5.
FIG. 7 is an explanatory view showing a still even another example of the print pattern used for applying the fine liquid droplets in the step 1.
FIG. 8 shows photographs of a sample taken from an upper surface side thereof before and after being subjected to a skin treatment in Example 13.
FIG. 9 shows photographs of a sample taken from an upper surface side thereof before and after being subjected to a skin treatment in Comparative Example 2.
FIG. 10 shows photographs of a sample taken from an upper surface side thereof before and after being subjected to a skin treatment in Example 42.
FIG. 11 is an explanatory view showing a further still even another example of the print pattern used for applying the fine liquid droplets in the step 1.
FIG. 12 is an explanatory view showing a yet further still even another example of the print pattern used for applying the fine liquid droplets in the step 1.
FIG. 13 is an explanatory view showing a yet further still even another example of the print pattern used for applying the fine liquid droplets in the step 1.
FIG. 14 shows photographs of a sample taken from an upper surface side thereof before and after being subjected to a skin treatment in Example 57.

### DETAILED DESCRIPTION OF THE INVENTION

Kinds of wrinkles are classified, according to causes for emergence of wrinkles and features of the wrinkles emerged, into cuticle type wrinkles, such as fine wrinkles, tiny wrinkles, etc.; dermis type wrinkles, such as ultraviolet-induced wrinkles, large wrinkles, etc.; facial muscle type wrinkles, such as wrinkles made along facial muscles. For this reason, it has been required to provide a method of improving various kinds of wrinkles in a non-invasive manner without damage to natural appearance of skin.

In addition, among various regions of a human face, the thickness of the skin, in particular, in regions around eyes, is small, and the regions around eyes tend to be well moved in association with blinks of the eyes, so that the wrinkles in such regions are increased with age. In particular, fine wrinkles made at an outer corner of the eye give a strong impression of aging. Therefore, there is an increasing demand for a non-invasive method of improving wrinkles in the delicate regions, such as the outer corner of the eye.

However, in the technologies of the Patent Literature 1, the skin external preparation for wrinkle improvement using a specific polymer having a low film shrinkage rate is applied to wrinkles to fill the wrinkles with a non-shrinkable film. Therefore, it will be difficult to apply the technologies of the Patent Literature 1 to delicate regions of skin having a thin skin thickness, such as skin regions around eyes.

In the technologies of the Patent Literature 2, the wrinkle smoothing effect can be attained to a certain extent. However, since the wrinkle smoothing agent used therein is applied to the skin by the method of spreading the liquid or past-like composition over the skin by human fingers, a spatula or the like, the wrinkle improving effect still has a room for further improvement thereof.

In the technologies of the Patent Literature 3, it is described that the aforementioned applicator assists in applying the skin smoothing composition in the form of a uniform film having a desired thickness which adheres to and contracts skin to smooth and flatten wrinkles and texture imperfections. However, since the method described in the Patent Literature 3 is the method in which the aforementioned composition is applied in the form of the uniform film to the skin through the flock fibers with which the head of the applicator is coated, there exists limitation to the region of the skin to which the composition can be applied, so that it is not possible to attain a sufficient wrinkle improving effect.

In addition, the injection treatment with a filler as the procedure of the cosmetic surgery has also been used for formation of eye bags. However, the procedure has been sometimes accompanied with difficulty in shape control owing to features of the regions to be treated, and therefore has also posed problems, such as a large physical burden on practitioners.

For this reason, it has been required that the changes in external shape of the skin, such as flattening of unevenness on a surface of the skin, impartation of a three-dimensional feel that makes unevenness on the surface of the skin conspicuous, as well as impartation of a resilient feel on the surface of the skin, lifting-up of a part of the surface of the skin, etc., are finely controlled in a non-invasive manner without damage to natural appearance of the skin.

The present invention relates to a skin treatment method that is capable of finely controlling an external shape of skin in a non-invasive manner without damage to natural appearance of the skin, and a makeup method using the skin treatment method.

The present inventors have found that by conducting a skin treatment method including the step of applying fine liquid droplets of a liquid composition containing a polymer and a volatile solvent to skin and the step of drying the fine liquid droplets applied to the skin in the previous step to contract the skin in association with the drying, in which the deformation ratio of the polymer contained in the liquid composition as measured by a specific method is controlled to a predetermined range, and the mass of the fine liquid droplets applied to the skin as measured per one droplet thereof is controlled to a predetermined range, it is possible to finely control an external shape of the skin in an non-invasive manner without damage to natural appearance of the skin.

That is, the present invention relates to the following aspects [1] to [3].
[1] A skin treatment method including:
   Step 1: applying fine liquid droplets of a liquid composition containing a polymer A and a volatile solvent B to skin; and
   Step 2: drying the fine liquid droplets applied to the skin in the step 1 to contract the skin in association with the drying, in which:
      a deformation ratio of the polymer A as measured by the following method (I) is not less than 0.3 and not more than 1; and
      a mass of the fine liquid droplets applied to the skin in the step 1 as measured per one droplet thereof is not less than 0.001 µg and not more than 100 µg,
      Method (I): dissolving the polymer A in the volatile solvent B to prepare a polymer solution having a concentration of 10% by mass; applying 0.005 g of the thus prepared polymer solution to a region of a surface of a polyethylene sheet having a width of 20 mm, a length of 100 mm and a thickness of 0.03 mm, the region having a width of 20 mm and a length of 50 mm which extends from an end of a short side of the polyethylene sheet along a length direction thereof; and drying the polymer solution applied to the polyethylene sheet in a constant temperature oven set at 40 °C for 10 minutes to prepare the polyethylene sheet with a polymer film,
      in which when a length of a portion of the polyethylene sheet to which the polymer solution is applied is represented by L1, and a length of a portion of the polyethylene sheet with the polymer film which undergoes deformation by drying the polymer solution applied thereto is represented by L2, a ratio of L2 to L1 [L2/L1] is defined as the deformation ratio of the polymer A.
[2] A makeup method using the skin treatment method according to the above aspect [1] for improving wrinkles on skin.
[3] A makeup method using the skin treatment method according to the above aspect [1] for forming undulation on skin.

In accordance with the present invention, it is possible to provide a skin treatment method that is capable of finely controlling an external shape of skin in an non-invasive manner without damage to natural appearance of the skin, and a makeup method using the skin treatment method.

### [Skin treatment method]

The skin treatment method of the present invention includes:
Step 1: applying fine liquid droplets of a liquid composition containing a polymer A and a volatile solvent B to skin; and
Step 2: drying the fine liquid droplets applied to the skin in the step 1 to contract the skin in association with the drying, in which:
   a deformation ratio of the polymer A as measured by the following method (I) is not less than 0.3 and not more than 1; and
   a mass of the fine liquid droplets applied to the skin in the step 1 as measured per one droplet thereof is not less than 0.001 µg and not more than 100 µg,
   Method (I): dissolving the polymer A in the volatile solvent B to prepare a polymer solution having a concentration of 10% by mass; applying 0.005 g of the thus prepared polymer solution to a region of a surface of a polyethylene sheet having a width of 20 mm, a length of 100 mm and a thickness of 0.03 mm, the region having a width of 20 mm and a length of 50 mm which extends from an end of a short side of the polyethylene sheet along a length direction thereof; and drying the polymer solution applied to the polyethylene sheet in a constant temperature oven set at 40 °C for 10 minutes to prepare the polyethylene sheet with a polymer film,
   in which when a length of a portion of the polyethylene sheet to which the polymer solution is applied is represented by L1, and a length of a portion of the polyethylene sheet with the polymer film which undergoes deformation by drying the polymer solution applied thereto is represented by L2, a ratio of L2 to L1 [L2/L1] is defined as the deformation ratio of the polymer A.

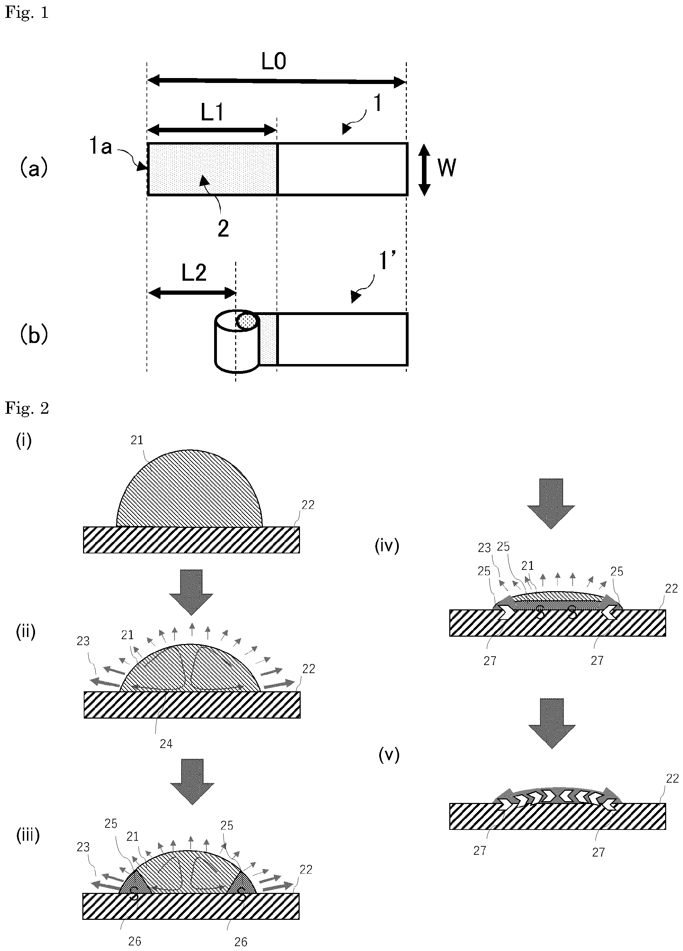

Meanwhile, the length L1 of the portion of the polyethylene sheet to which the polymer solution is applied and the length L2 of the portion of the polyethylene sheet with the polymer film may be apparently recognized by referring to FIG. 1.

Incidentally, the term "skin treatment method" as used in the present invention means the "method for controlling an external shape of skin". The concept of the "control of an external shape of skin" includes the change in external shape of the skin by flattening unevenness on the surface of the skin, the change in external shape of the skin by imparting a three-dimensional feel to the skin by making unevenness on the surface of the skin conspicuous, as well as the change in external shape of the skin by imparting a resilient feel onto the surface of the skin or lifting-up a part of the surface of the skin, and also includes, for example, the change in external shape of the skin by reducing wrinkles to have a rejuvenating effect of allowing an appearance of a person to look younger than a real age, or the change in external shape of the skin by making wrinkles conspicuous to have an aging effect of allowing an appearance of a person to look older than a real age.

The term "wrinkles on skin" as used in the present invention means unevenness and streaks that appear on the surface of the skin. The wrinkles on the skin are liable to appear around the eyes, around the mouth, and in forehead, neck, etc. Small streaks are fine wrinkles, and large streaks are large wrinkles. The small wrinkles are caused at outer corners of the eyes, inner corners of the eyes, etc., and the large wrinkles are caused by a flow of pores on the nasolabial fold, cheeks, and the like.

The term "improvement of wrinkles on skin" as used in the present invention means the method of drying the fine liquid droplets of the aforementioned liquid composition which are applied to the skin to contract the skin around the wrinkles in association with the drying, whereby recesses on the skin which form the wrinkles are flattened, as described in detail hereinbelow. That is, the "improvement of wrinkles on skin" means the method of physically stretching the wrinkles to flatten unevenness on the surface of the skin. Thus, the "improvement of wrinkles on skin" includes a concept that is different from, for example, the method in which a cosmetic material and the like are applied to the skin to fill unevenness on the surface of the skin therewith or the method of making the wrinkles optically inconspicuous.

In addition, the term "formation of undulation on skin" as used in the present invention means the method of drying the fine liquid droplets of the aforementioned liquid composition which are applied to the skin to contract the skin in association with the drying, whereby the skin tissue is three-dimensionally lifted up to make unevenness on the surface of the skin conspicuous, as described hereinbelow.

Moreover, the term "improvement of sagging of skin" as used herein means the method of drying the fine liquid droplets of the aforementioned liquid composition which are applied to the skin to contract the skin in association with the drying, whereby a resilient feel is imparted to the skin, or a part of the surface of the skin is raised to lift up the sagging skin, as described hereinbelow.

In accordance with the present invention, it is possible to provide a skin treatment method that is capable of finely controlling an external shape of skin in an non-invasive manner without damage to natural appearance of the skin. The reason why the aforementioned advantageous effects can be attained by the present invention is considered as follows, though it is not clearly determined yet.

Here, an example of the mechanism of occurrence of the skin contraction according to the present invention is explained by referring to FIG. 2.

In the present invention, the polymer A is present in the form of a solution prepared by dissolving the polymer A in the volatile solvent B, in the liquid composition. When the fine liquid droplets 21 of the aforementioned liquid composition are applied to the skin 22 in the step 1 as shown in FIG. 2(i), volatilization of the volatile solvent B contained in the fine liquid droplets 21 is initiated in the drying process of the fine liquid droplets 21 in the step 2 as shown in FIG. 2(ii) (the arrows 23 in FIG. 2 indicate a manner of volatilization of the solvent). The volatile solvent B is volatilized preferentially from an end portion of each fine liquid droplet 21 according to the temperature of a portion of the skin which comes into contact with the end portion of the fine liquid droplet 21, so that there occurs the difference in surface tension between the end portion and the central portion of the fine liquid droplet. As a result, a Marangoni convection 24 is caused in the respective fine liquid droplets 21, so that the concentration of the polymer A in the end portion of the fine liquid droplet 21 is enhanced, and the end portion of the fine liquid droplet 21 is cured, i.e., solidified owing to precipitation of the polymer A, earlier than the central portion of the fine liquid droplet 21 as shown in FIG. 2 (iii), whereby the skin 26 located below a cured coating film of the end portion 25 of the fine liquid droplet 21 is fixed. After forming the cured coating film of the end portion 25, the volatile solvent B is also volatilized from the central portion of the fine liquid droplet 21, so that there is caused contraction of the coating film. At this time, as shown in FIG. 2 (iv), the cured coating film of the end portion 25 is fixed to the skin, and further since such a requirement that the deformation ratio of the polymer A as measured by the aforementioned method (I) lies within the predetermined range can be satisfied, a contraction stress 27 is applied to the skin along with the contraction of the cured coating film owing to volatilization of the volatile solvent B. As a result, as shown in FIG. 2 (v), it is considered that the contraction stress 27 is applied to the skin at an end point of the drying process of the fine liquid droplet 21 to exert a high contraction effect against the skin.

In the present invention, by controlling the mass of the fine liquid droplets applied to the skin in the step 1 as measured per one droplet thereof to the predetermined range, it is possible to increase the proportion of the end portion of the respective fine liquid droplets on the basis of the amount of the liquid composition applied as compared to the case where the same amount of the liquid composition as described above is applied and spread over the skin by human fingers, a spatula or the like, and promote volatilization of the volatile solvent B from the end portion of the respective fine liquid droplets in the step 2. For this reason, it is considered that since the drying velocity of the fine liquid droplets applied to the skin is increased, the effect of contraction of the skin can be improved.

In addition, in the present invention, the coating films formed of the respective fine liquid droplets can be discontinuously formed on the surface of the skin in the step 1. Therefore, it is possible to suppress occurrence of shiny smooth feel over a whole surface of the skin treated. For example, even in the case where the content of the polymer A in the liquid composition is small, and the drying velocity of the fine liquid droplets applied is low, occurrence of mirror reflection of light on the surface of the skin treated is inhibited, so that it is possible to improve capability of exerting a contraction action of the skin without damage to natural appearance of the skin.

Also, in the case where the liquid composition is applied and spread over the skin by human fingers, a spatula or the like, and for example, in the case where the content of the polymer A in the liquid composition is small, and the drying velocity of the liquid composition applied to the skin is high, fine concavo-convex unevenness tends to be generated in the coating film owing to Benard cells in association with occurrence of the aforementioned Marangoni convection, in some cases.

On the other hand, in the present invention, by controlling the mass of the fine liquid droplets applied to the skin in the step 1 as measured per one droplet thereof to the predetermined range, the size of the respective Benard cells generated can be restricted, and it is therefore possible to suppress occurrence of the fine concavo-convex unevenness in the coating film.

Moreover, by discontinuously forming the coating films each formed of the fine liquid droplet on the surface of the skin, it is possible not only to inhibit occurrence of mirror reflection of light on the surface of the skin after being treated and enhance the blurring effect owing to scattering of light, but also to impart a transparent feel to the skin.

The skin treatment method of the present invention is preferably applied to a beauty care method or a makeup method.

For example, when applying the skin treatment method of the present invention to a beauty care method or makeup method for improving wrinkles on the skin, the skin in the peripheral portions of the wrinkles can be contracted owing to the mechanism of occurrence of the skin contraction, so that the recesses on the skin which form the wrinkles can be flattened to make the wrinkles inconspicuous.

In addition, when applying the skin treatment method of the present invention to a beauty care method or makeup method for forming undulation on the skin, the skin portion where it is desired to make unevenness on the surface of the skin conspicuous can be contracted owing to the mechanism of occurrence of the skin contraction, so that the skin tissue thereof can be lifted up to form undulation on the skin to thereby impart a good three-dimensional feel to the skin.

Thus, when suitably selecting parts of human body to which the fine liquid droplets of the liquid composition are applied, it is possible to well control unevenness on the surface of the skin.

In addition, in the present invention, since the end portion of the respective fine liquid droplets is cured earlier than the central portion thereof as described above, occurrence of such a so-called coffee ring phenomenon that the polymer A is accumulated on the end portion of the coating film can be inhibited. Therefore, by properly controlling the mass of the fine liquid droplets applied as measured per one droplet thereof, the thickness of the end portion of the coating film can be reduced relative to the other portions thereof, and the polymer A can be more accumulated in the central portion of the coating film. For this reason, it is considered that by properly selecting parts of human body to which the fine liquid droplets are applied, the central portion of the respective fine liquid droplets undergoes contraction action during the drying process thereof with the end portion of the respective fine liquid droplets being fixed, so that the effect of lifting-up the skin can also be exhibited.

Accordingly, by using the skin treatment method of the present invention, it is possible not only to improve wrinkles on the skin and form undulation on the skin, but also to improve sagging of the skin. Therefore, the skin treatment method of the present invention can be applied to the procedures for beauty care, such as impartation of a resilient feel to the skin or lift-up of a part of the skin, etc.

Thus, in the present invention, by appropriately selecting parts of human body to which the fine liquid droplets of the liquid composition are to be applied in the step 1 and allowing the skin of the respective parts of human body to undergo contraction in the step 2, it is possible to promote and finely control deformation of an external shape of the skin in a non-invasive manner. Therefore, the skin treatment method of the present invention can be used as a non-invasive cosmetic surgery technology, or as a beauty care method for conducting non-invasive cosmetic treatments.

### (Step 1)

The step 1 is the step of applying the fine liquid droplets of the liquid composition containing the polymer A and the volatile solvent B to skin.

In the present invention, the method of applying the fine liquid droplets of the liquid composition to the skin in the step 1 includes not only the method of directly applying the fine liquid droplets of the liquid composition to the surface of the skin, but also the method of applying the fine liquid droplets of the liquid composition to the skin on which a cosmetic material, for example, such as a solution, an emulsion, a cream, a sheet, etc., has been previously applied, through the cosmetic material.

### <Liquid Composition>

The liquid composition used in the present invention contains the polymer A and the volatile solvent B.

The liquid composition is preferably maintained in a liquid state under 1 atm at 25 °C from the viewpoint of finely controlling an external shape of the skin without damage to natural appearance thereof.

### (Polymer A)

The deformation ratio (L2/L1) of the polymer A used in the present invention as measured by the aforementioned method (I) is not less than 0.3 and not more than 1. The method of measuring the deformation ratio (L2/L1) of the polymer A by the method (I) is specifically described in Examples below. As the value of the deformation ratio (L2/L1) is increased, the amount of contraction of the coating film containing the polymer A owing to drying thereof becomes larger. The upper limit of the deformation ratio (L2/L1) is 1.

The deformation ratio (L2/L1) of the polymer A as measured by the aforementioned method (I) can be suitably controlled by the combination of the polymer A and the volatile solvent B.

From the viewpoint of finely controlling an external shape of the skin without damage to natural appearance thereof, the deformation ratio (L2/L1) of the polymer A is not less than 0.3, preferably not less than 0.5, more preferably not less than 0.6, even more preferably not less than 0.7, further even more preferably not less than 0.8, still further even more preferably not less than 0.85, yet still further even more preferably not less than 0.9 and yet still further even more preferably not less than 0.95, and is also not more than 1.

The polymer A is not particularly limited as long as it is capable of satisfying the aforementioned deformation ratio.

In the present invention, since the liquid composition is applied in the form of fine liquid droplets to the skin in the step 1, it is possible to discontinuously form contractible coating films on the surface of the skin. Since no coating films are formed on the region of the skin on which any fine liquid droplets of the liquid composition are not applied in the step 1, the region of the skin is preferentially deformed by the contraction stress applied thereto during the drying process in the step 2. For this reason, the coating films undergoing the contraction action tend to hardly suffer from fracture or breakage, so that the polymer A having a comparatively high contraction force can be used for the aforementioned liquid composition. Thus, in the present invention, a high degree of freedom of designing can be attained with regard to the kind of polymer A, the content of the polymer A in the aforementioned liquid composition, the amount of the fine liquid droplets applied per a unit area, etc.

Examples of the basic skeleton of the polymer A include a condensation-based polymer, such as a polyurethane, a polyester, a polyether, a polyurea, etc.; a vinyl-based polymer obtained by addition-polymerization of a vinyl-based monomer, such as a vinyl compound, a vinylidene compound, a vinylene compound, a cyclic olefin, etc.; a silicone-based polymer; and the like. Of these polymers, from the viewpoint of finely controlling an external shape of the skin without damage to natural appearance thereof, preferred is at least one polymer selected from the group consisting of a vinyl-based polymer and a silicone-based polymer.

As the vinyl-based monomer constituting the aforementioned vinyl-based polymer used as the polymer A, there may be used those vinyl-based monomers containing an anionic group or a cationic group.

Examples of the anionic group contained in the vinyl-based monomer include groups that are capable of releasing hydrogen ions upon dissociation thereof to allow the polymer to exhibit acidity, such as a carboxy group (-COOM), a sulfonic acid group (-SOsM), a phosphoric acid group (-OPO₃M₂), etc., or dissociated ion forms of these groups (such as -COO⁻, -SO₃⁻, -OPO₃²⁻ and -OPO₃⁻M); and the like. In the aforementioned chemical formulae, M represents a hydrogen atom, an alkali metal, ammonium or an organic ammonium.

Examples of the anionic group-containing vinyl-based monomer include acrylic acid, methacrylic acid, maleic acid, styrenesulfonic acid, and the like.

Examples of the cationic group contained in the vinyl-based monomer include a protonic acid salt of a primary, secondary or tertiary amino group, a quaternary ammonium group, and the like.

Examples of the cationic group-containing vinyl-based monomer include an *N*,*N*-dialkylaminoalkyl (meth)acrylate containing an *N,N-*dialkylaminoalkyl group having a total carbon number of not less than 4 and not more than 14, such as N,N dimethylaminoethyl (meth)acrylate, etc.; an *N*,*N*-dialkylaminoalkyl (meth)acrylamide containing an N,N dialkylaminoalkyl group having a total carbon number of not less than 4 and not more than 14, such as N,N-dimethylaminopropyl (meth)acrylamide, etc.; and the like.

From the viewpoint of finely controlling an external shape of the skin without damage to natural appearance thereof, the aforementioned vinyl-based polymer is preferably a vinyl-based polymer containing a repeating unit derived from the anionic group-containing vinyl-based monomer, more preferably a vinyl-based polymer containing a repeating unit derived from at least one vinyl-based monomer selected from the group consisting of acrylic acid, methacrylic acid, maleic acid, styrenesulfonic acid, and salts of these acids, and even more preferably at least one vinyl-based polymer selected from the group consisting of polymethacrylic acid and poly(sodium styrenesulfonate).

In the case where the aforementioned vinyl-based polymer contains the repeating unit derived from the anionic group-containing vinyl-based monomer, the vinyl-based polymer may be in the form of a copolymer that also contains a repeating unit derived from the other monomer in addition to the repeating unit derived from the anionic group-containing vinyl-based monomer. However, from the viewpoint of finely controlling an external shape of the skin without damage to natural appearance thereof, the vinyl-based polymer is preferably in the form of a homopolymer of the anionic group-containing vinyl-based monomer.

The weight-average molecular weight Mw of the aforementioned vinyl-based polymer is preferably not less than 10,000, more preferably not less than 30,000 and even more preferably not less than 50,000, and is also preferably not more than 700,000, more preferably not more than 500,000 and even more preferably not more than 300,000. The aforementioned weight-average molecular weight Mw may be measured by a gel permeation chromatography (GPC) using polystyrenes as reference standard substance, more specifically, by the method described in Examples below.

The aforementioned vinyl-based polymer may be either an appropriately synthesized product or a commercially available product.

In the case where the vinyl-based polymer is a synthesized product, the synthesized product may be produced, fro example, by subjecting the aforementioned anionic group-containing vinyl-based monomer, if required together with the other monomers, to addition polymerization reaction in the presence of a polymerization initiator, etc., by conventionally known methods.

Examples of the commercially available product of the aforementioned vinyl-based polymer include poly(sodium styrenesulfonate) available from Alfa Aesar, and the like.

The aforementioned silicone-based polymer used as the polymer A is preferably a silicone polymer having a silicone structure.

The "silicone structure" as used in the present specification means a structure represented by the following general formula (I): 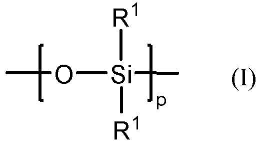 In the general formula (I), R¹ groups are each independently a hydrocarbon group having not less than 1 and not more than 12 carbon atoms, and p is an integer of not less than 1.

R¹ is preferably an alkyl group having not less than 1 and not more than 12 carbon atoms or an aryl group having not less than 6 and not more than 12 carbon atoms, more preferably an alkyl group having not less than 1 and not more than 12 carbon atoms or a phenyl group, even more preferably an alkyl group having not less than 1 and not more than 3 carbon atoms, and further even more preferably a methyl group, from the viewpoint of finely controlling an external shape of the skin without damage to natural appearance thereof as well as from the viewpoint of improving versatility.

The aforementioned silicone polymer is not particularly limited as long as it has at least one silicon structure therein. The silicone polymer may be a polymer constituted of the silicon structure only. However, from the viewpoint of adjusting the aforementioned deformation ratio to the predetermined range and finely controlling an external shape of the skin without damage to natural appearance thereof, the silicone polymer is preferably a polymer having the silicone structure as a part thereof.

In the case where the silicone polymer is a polymer having the silicone structure as a part thereof, the silicone structure may be present in either a main chain or a side chain of the polymer. From the viewpoint of adjusting the aforementioned deformation ratio to the predetermined range and finely controlling an external shape of the skin without damage to natural appearance thereof, the silicone structure is preferably present in a side chain of the polymer.

In the case where the silicone structure is present in a main chain of the polymer, the bonding configuration of the silicone structure in the main chain of the polymer is not particularly limited. For example, the silicone structure may be present in a terminal end of the main chain of the polymer, or the silicone polymer may be in the form of a copolymer formed by bonding the silicone structures in a block form or a random form in the main chain of the polymer. In addition, as the silicone polymer, there may also be used a polymer graft-modified by a compound having the silicone structure.

The aforementioned silicone polymer is preferably a silicone-modified polymer. Specific examples of the silicone-modified polymer include norbornane structure-containing silicone-modified polymers, such as silicone-modified polynorbornene, etc.; silicone-modified pullulan; silicone structure-containing silicic acid compounds, such as a trialkylsiloxysilicic acid, a fluorine-modified alkylsiloxysilicic acid, a phenyl-modified alkylsiloxysilicic acid, etc.; silicone dendrimers: and the like.

### [Norbornane Structure-Containing Silicone-Modified Polymer]

The term "norbornane structure" as used in the present specification means the structure represented by the following formula. The norbornane structure-containing silicone-modified polymer may be a silicone-modified polymer having a norbornane structure represented by the formula: 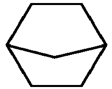 in an arbitrary site in the polymer.

Examples of the norbornane structure-containing silicone-modified polymer include a polymer containing a repeating unit represented by the following general formula (1) or (2).

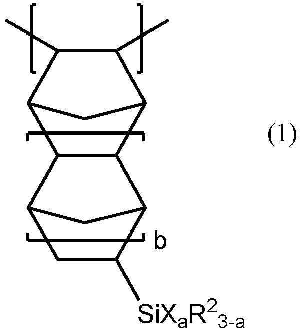

In the general formula (1), R² groups are each independently an alkyl group having not less than 1 and not more than 12 carbon atoms; X is a group represented by the following formula (i); a is an integer of not less than 1 and not more than 3; and b is an integer of not less than 0 and not more than 2.

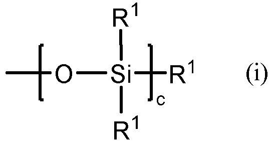

In the general formula (i), R¹ groups are the same as described above; and c is an integer of not less than 1 and not more than 5.

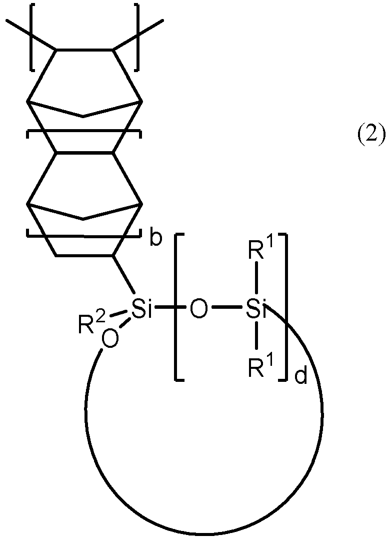

In the general formula (2), R¹, R², and b are the same as described above; and d is an integer of not less than 2 and not more than 5.

In the general formula (1), R² groups are each independently an alkyl group having not less than 1 and not more than 12 carbon atoms, and from the viewpoint of finely controlling an external shape of the skin without damage to natural appearance thereof as well as from the viewpoint of improving versatility, R² is preferably an alkyl group having not less than 1 and not more than 5 carbon atoms, more preferably a methyl group, an ethyl group, an n-propyl group, a butyl group or a pentyl group, and even more preferably a methyl group.

In the general formula (i), R¹ is preferably an alkyl group having not less than 1 and not more than 12 carbon atoms or an aryl group having not less than 6 and not more than 12 carbon atoms, more preferably an alkyl group having not less than 1 and not more than 12 carbon atoms or a phenyl group, even more preferably an alkyl group having not less than 1 and not more than 3 carbon atoms, and further even more preferably a methyl group, from the viewpoint of finely controlling an external shape of the skin without damage to natural appearance thereof as well as from the viewpoint of improving versatility. In the general formula (i), c is an integer of not less than 1 and not more than 5, and from the viewpoint of improving versatility, c is preferably 1. That is, X is preferably a trimethylsiloxy group.

In the general formula (1), a is an integer of not less than 1 and not more than 3, and the aforementioned silicone-modified polymer may also be, for example, a polymer in which a repeating unit of a = 2 and a repeating unit of a = 3 are present as a mixture. From the viewpoint of improving versatility, a is preferably 3. In the general formula (1), b is an integer of not less than 0 and not more than 2, and from the same viewpoint as described above, b is preferably 0, 1, or a combination thereof, and more preferably 0.

In the general formula (2), R¹, R², and b are the same as those described above, and d is an integer of not less than 2 and not more than 5, and from the viewpoint of improving versatility, d is preferably 2. From the same viewpoint as described above, in the cyclic silicone structure in the general formula (2), it is preferred that R¹ and R² are each a methyl group, and d is 2 or 3. That is, the cyclic silicone structure in the general formula (2) is preferably the structure represented by the following formula (2-1) or (2-2).

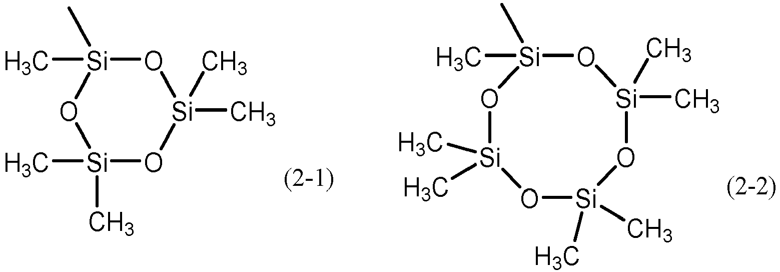

From the viewpoint of finely controlling an external shape of the skin without damage to natural appearance thereof as well as from the viewpoint of improving versatility, the proportion of the repeating unit represented by the general formula (1) or (2) in the norbornane structure-containing silicone-modified polymer on the basis of the total number of the repeating units contained in the polymer is preferably not less than 10%, more preferably not less than 30% and even more preferably not less than 50%, and is also preferably not more than 100%, more preferably not more than 95%, even more preferably not more than 90% and further even more preferably not more than 70%.

The norbornane structure-containing silicone-modified polymer may further contain a repeating unit represented by the following general formula (3) in addition to the repeating unit represented by the general formula (1) or (2).

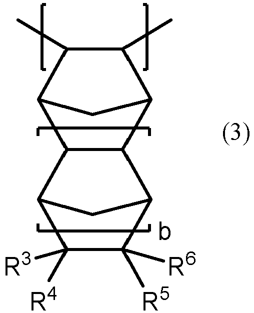

In the general formula (3), R³ to R⁶ are each independently a hydrogen atom, a halogen atom, a substituent group selected from the group consisting of an alkyl group, an alkenyl group, a cycloalkyl group, an aryl group, an alkoxy group, an aryloxy group and a halogenated hydrocarbon group, which groups each have not less than 1 and not more than 10 carbon atoms, an oxetanyl group, an alkoxycarbonyl group, a polyoxyalkylene group, a polyglyceryl group, or an alkoxysilyl group. Any two groups selected from R³ to R⁶ may be bonded to each other to form an alicyclic structure, an aromatic ring structure, a carboimide group, or an acid anhydride group. In the general formula (3), b is the same as described above.

From the viewpoint of finely controlling an external shape of the skin without damage to natural appearance thereof as well as from the viewpoint of improving versatility, the proportion of the repeating unit represented by the general formula (3) in the norbornane structure-containing silicone-modified polymer on the basis of the total number of the repeating units contained in the polymer is preferably not more than 90%, more preferably not more than 70% and even more preferably not more than 50%. In addition, in the case where the silicone-modified polymer contains the repeating unit represented by the general formula (3), the proportion of the repeating unit represented by the general formula (3) on the basis of the total number of the repeating units contained in the polymer is preferably not less than 5%, more preferably not less than 10% and even more preferably not less than 30%.

The ratios of the numbers of the repeating units represented by the general formulae (1) to (3) in the norbornane structure-containing silicone-modified polymer can be determined by the ¹H-NMR measurement.

The norbornane structure-containing silicone-modified polymer is preferably a silicone-modified polynorbornene, and more preferably a silicone-modified polynorbornene represented by the following formula (4).

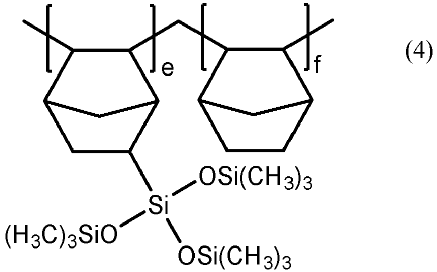

In the formula (4), e and f are the numbers of the respective repeating units, and are each independently an integer of not less than 1.

From the viewpoint of finely controlling an external shape of the skin without damage to natural appearance thereof, the molar ratio of e to f (e/f) in the formula (4) is preferably from 20/80 to 90/10, more preferably from 30/70 to 80/20, even more preferably from 50/50 to 70/30, further even more preferably from 55/45 to 70/30, and still further even more preferably from 55/45 to 65/35.

From the viewpoint of finely controlling an external shape of the skin without damage to natural appearance thereof as well as from the viewpoint of improving versatility, the number average molecular weight Mn of the norbornane structure-containing silicone-modified polymer is preferably not less than 50,000, more preferably not less than 100,000 and even more preferably 200,000, and is also preferably not more than 2,000,000, more preferably not more than 1,500,000, even more preferably not more than 800,000, further even more preferably not more than 600,000 and still further even more preferably not more than 400,000.

The number average molecular weight Mn of the polymer can be measured by a gel permeation chromatography (GPC) using polystyrenes as a reference standard substance, and more specifically, it may be measured by the method described in Examples below.

The norbornane structure-containing silicone-modified polymer may be obtained, for example, by subjecting a silicone-modified cyclic olefin monomer that contains a norbornane structure or is capable of forming the norbornane structure, to addition polymerization by conventionally known methods.

For example, the norbornane structure-containing silicone-modified polymer containing the repeating unit represented by the general formula (1) or (2) can be obtained by subjecting a cyclic olefin monomer represented by the following general formula (1a) or (2a) to addition polymerization. Furthermore, in the addition polymerization, a cyclic olefin monomer represented by the following general formula (3a) may also be copolymerized with the aforementioned monomer.

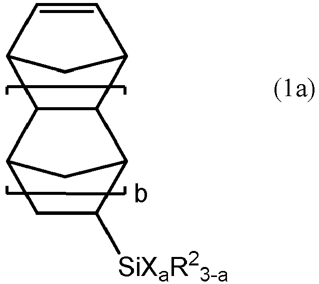

In the general formula (1a), R², X, a and b are the same as those described above.

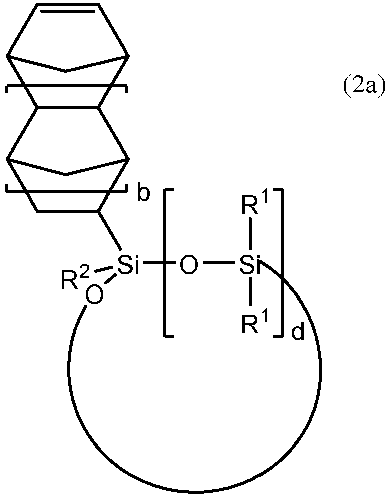

In the general formula (2a), R¹, R², b and d are the same as those described above.

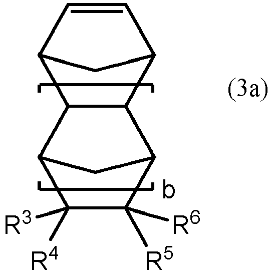

In the general formula (3a), R³ to R⁶ and b are the same as those described above.

In the case where the cyclic olefin monomer represented by the general formula (3a) is copolymerized, from the viewpoint of finely controlling an external shape of the skin without damage to natural appearance thereof as well as from the viewpoint of improving versatility, the amount of the cyclic olefin monomer of the general formula (3a) used is preferably not more than 90 mol%, more preferably not more than 70 mol% and even more preferably not more than 50 mol%, and is also preferably not less than 5 mol%, more preferably not less than 10 mol% and even more preferably not less than 30 mol%, on the basis of 100 mol% of the whole monomers used in the polymerization.

The silicone-modified polynorbornene represented by the aforementioned formula (4) may be obtained by subjecting tris(trimethylsiloxy)silylnorbornene represented by the following formula (4a) and norbornene to addition copolymerization.

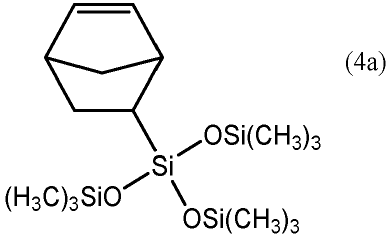

From the viewpoint of finely controlling an external shape of the skin without damage to natural appearance thereof, the molar ratio of tris(trimethylsiloxy)silylnorbornene to norbornene [tris(trimethylsiloxy)silylnorbornene/norbornene] in the copolymer thereof is preferably not less than 20/80, more preferably not less than 30/70, even more preferably not less than 50/50 and further even more preferably not less than 55/45, and is also preferably not more than 90/10, more preferably not more than 80/20, even more preferably not more than 70/30 and further even more preferably not more than 65/35.

Incidentally, although the repeating units represented in the general formulae (1) to (3) and the formula (4) all represent a 2,3-addition structural unit of the cyclic olefin monomer that is the raw material monomer of the polymer, a 2,7-addition structural unit of the cyclic olefin monomer due to the addition polymerization thereof may be included therein.

As the norbornane structure-containing silicone-modified polymer, a (norbornene/tris(trimethylsiloxy)silylnorbornene) copolymer is preferred, and the copolymer is a compound expressed by NORBORNENE/TRIS(TRIMETHYLSILOXY)SILYLNORBORNENE COPOLYMER according to the INCI Name (International Cosmetic Ingredient Dictionary and Handbook, 16th Edition, Volume 2, 2016, p. 2274).

Examples of a commercially available product of the norbornane structure-containing silicone-modified polymer include "NBN-30-ID" (an isododecane solution of a (norbornene/tris(trimethylsiloxy) silylnorbornene) copolymer) available from Shin-Etsu Chemical Co., Ltd., and the like.

### [Silicone-Modified Pullulan]

As the silicone-modified pullulan, there may be mentioned those pullulans having a silicone structure in a side chain thereof. More specifically, from the viewpoint of finely controlling an external shape of the skin without damage to natural appearance thereof as well as from the viewpoint of improving versatility, preferred is a silicone-modified pullulan that is produced by substituting at least a part of hydrogen atoms in OH groups contained in pullulan with the group represented by the following general formula (5).

-Z¹-SiXₐR²₃₋ₐ (5)

In the general formula (5), Z¹ is a single bond or a divalent organic group, and R², X and a are the same as those described above. From the same viewpoint as described above, X is preferably a trimethylsiloxy group, and a is preferably 3.

In the general formula (5), from the same viewpoint as described above, Z¹ is preferably a divalent organic group, more preferably a divalent group represented by the following general formula (5-1) or (5-2), and even more preferably a divalent group represented by the following general formula (5-2).

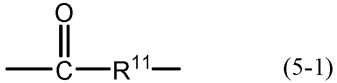

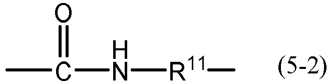

In the general formulae (5-1) and (5-2), R¹¹ is an alkylene group having not less than 1 and not more than 10 carbon atoms. Examples of the alkylene group as R¹¹ include a methylene group, an ethylene group, a trimethylene group, a propylene group, a butylene group, and the like. From the same viewpoint as described above, among these alkylene groups, preferred is an ethylene group, a trimethylene group or a propylene group, and more preferred is a trimethylene group or a propylene group.

Examples of commercially available products of the silicone-modified pullulan include "TSPL-30-ID" (an isododecane solution of tri(trimethylsiloxy)silylpropylcarbamic acid pullulan) and "TSPL-30-D5" (a cyclopentasiloxane solution of tri(trimethylsiloxy)silylpropylcarbamic acid pullulan) both available from Shin-Etsu Chemical Co., Ltd., and the like.

### [Silicone Structure-Containing Silicic Acid Compound]

As the silicone structure-containing silicic acid compound used in the present invention, there may be mentioned a silicate compound having a silicone structure in a terminal end thereof. Examples of the silicone structure-containing silicic acid compound include a trialkyl siloxysilicic acid, a fluorine-modified alkyl siloxysilicic acid, a phenyl-modified alkyl siloxysilicic acid, and the like.

From the viewpoint of finely controlling an external shape of the skin without damage to natural appearance thereof as well as from the viewpoint of improving versatility, as the alkyl group of the trialkyl siloxysilicic acid, preferred are alkyl groups having not less than 1 and not more than 10 carbon atoms, more preferred are alkyl groups having not less than 1 and not more than 4 carbon atoms, and even more preferred is a methyl group. Specific examples of the trialkyl siloxysilicic acid include trimethyl siloxysilicic acid.

As the fluorine-modified alkyl siloxysilicic acid, there may be mentioned those compounds formed by substituting at least a part of hydrogen atoms of the alkyl groups in the trialkyl siloxysilicic acid with a fluorine atom. Specific examples of the fluorine-modified alkyl siloxysilicic acid include trifluoropropyldimethyl siloxysilicic acid, trifluoropropyldimethyl/trimethyl siloxysilicic acid, and the like.

Examples of the phenyl-modified alkyl siloxysilicic acid include phenylpropyldimethyl siloxysilicic acid, phenylpropyldimethyl/trimethyl siloxysilicic acid, and the like.

Among these silicone structure-containing silicic acid compounds, from the viewpoint of finely controlling an external shape of the skin without damage to natural appearance thereof, preferred is at least one compound selected from the group consisting of the trialkyl siloxysilicic acid and the fluorine-modified alkyl siloxysilicic acid.

Examples of commercially available products of the silicone structure-containing silicic acid compound used herein include trimethyl siloxysilicic acids (solution) available from Shin-Etsu Chemical Co., Ltd., such as "KF-7312J", "KF-7312K", "KF-7312T", "KF-7312L", "X-21-5249", "X-21-5250", "KF-9021", "X-21-5595", "X-21-5616", "KF-9021L", "X-21-5249L", "X-21-5250L", etc.; "XS66-B8226" (a cyclopentasiloxane solution of trifluoropropyldimethyl/trimethyl siloxysilicic acid), "XS66-B8636" (a dimethicone solution of trifluoropropyldimethyl/trimethyl siloxysilicic acid) and "SilShine151" (phenylpropyldimethyl siloxysilicic acid) all available from Momentive Performance Materials Inc.; and the like.

### [Silicone Dendrimer]

Examples of the silicone dendrimer include vinyl- based polymers having a siloxane dendrimer structure at a side chain thereof. From the viewpoint of finely controlling an external shape of the skin without damage to natural appearance thereof as well as from the viewpoint of improving versatility, the siloxane dendrimer structure is preferably constituted of a group represented by the following general formula (6).

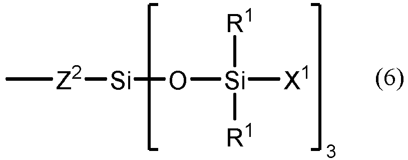

In the general formula (6), R¹ is the same as described above; Z² is a single bond or a divalent organic group; X¹ is a group represented by the following general formula (6-1) in the case of i = 1 wherein i represents a hierarchy of the group and is an integer of not less than 1 and not more than 10.

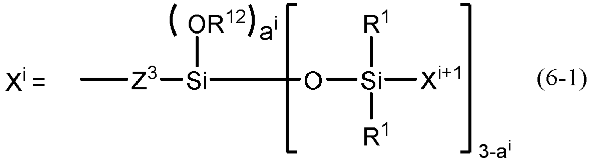

In the general formula (6-1), R¹ is the same as described above; R¹² is an alkyl group having not less than 1 and not more than 10 carbon atoms; Z³ is an alkylene group having not less than 2 and not more than 10 carbon atoms; Xⁱ⁺¹ is a hydrogen atom, an alkyl group having not less than 1 and not more than 10 carbon atoms, an aryl group or the group represented by the general formula (6-1); and aⁱ is an integer of not less than 0 and not more than 3.

In the general formula (6), Z² is a single bond or a divalent organic group. From the viewpoint of improving versatility, Z² is preferably a divalent organic group, and more preferably a divalent group represented by the following general formula (6-2), (6-3) or (6-4).

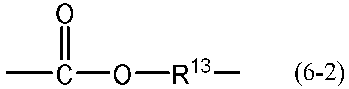

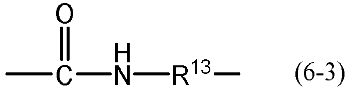

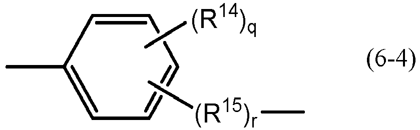

In the general formulae (6-2), (6-3) and (6-4), R¹³ is an alkylene group having not less than 1 and not more than 10 carbon atoms. Examples of the alkylene group as R¹³ include a methylene group, an ethylene group, a trimethylene group, a propylene group, a butylene group, and the like. Among these alkylene groups as R¹³, from the viewpoint of improving versatility, preferred is an ethylene group, a trimethylene group or a propylene group. R¹⁴ is an alkyl group having not less than 1 and not more than 10 carbon atoms. Examples of the alkyl group as R¹⁴ include a methyl group, an ethyl group, a propyl group, a butyl group, and the like. Among these alkyl groups as R¹⁴, from the same viewpoint as described above, preferred is a methyl group. R¹⁵ is an alkylene group having not less than 1 and not more than 10 carbon atoms. Examples of the alkylene group as R¹⁵ include a methylene group, an ethylene group, a trimethylene group, a propylene group, a butylene group, and the like. Among these alkylene groups as R¹⁵, from the same viewpoint as described above, preferred is an ethylene group. In the general formula (6-4), q is an integer of not less than 0 and not more than 4, and r is 0 or 1.

As the vinyl-based polymer having the aforementioned siloxane dendrimer structure in a side chain thereof, there may be mentioned a polymer containing a repeating unit derived from a monomer represented by the following general formula (7).

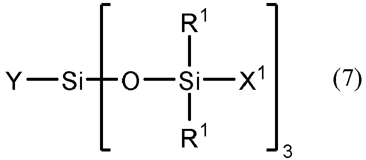

In the general formula (7), R¹ and X are the same as those described above, and Y is a group containing a vinyl bond. Examples of the group containing a vinyl bond include a vinyl group, a 2-acryloyloxyethyl group, a 3-acryloyloxypropyl group, a 2-methacryloyloxyethyl group, a 3-methacryloyloxypropyl group, a 4-vinylphenyl group, a 3-vinylphenyl group, a 4-(2-propenyl)phenyl group, a 3-(2-propenyl)phenyl group, a 2-(4-vinylphenyl)ethyl group, a 2-(3-vinylphenyl)ethyl group, an allyl group, and a 5-hexenyl group. Among these groups, from the viewpoint of improving versatility, preferred is a (meth)acryloyl group or a vinyl group, and more preferred is a (meth)acryloyl group.

The vinyl-based polymer having the aforementioned siloxane dendrimer structure in a side chain thereof may also contain a repeating unit derived from a vinyl-based monomer other than the monomer represented by the general formula (7). As the vinyl-based monomer, there may be mentioned those monomers that contain the group containing a vinyl bond and are monomers other than the monomer represented by the general formula (7). Examples of the vinyl-based monomer include (meth)acrylic acid, an alkyl (meth)acrylate, a hydroxyalkyl (meth)acrylate, an aromatic ring-containing (meth)acrylate, a fatty acid vinyl ester, (meth)acrylamide, styrene or derivatives thereof, and the like. These monomers may be used alone or in combination of any two or more thereof. Among these monomers, from the viewpoint of improving versatility, preferred are (meth)acrylic monomers, such as (meth)acrylic acid, an alkyl (meth)acrylate, a hydroxyalkyl (meth)acrylate, an aromatic ring-containing (meth)acrylate, etc.

The content of the repeating unit derived from the monomer represented by the general formula (7) in the vinyl-based polymer having the aforementioned siloxane dendrimer structure in a side chain thereof on the basis of the whole repeating units contained in the vinyl-based polymer is preferably not less than 0.1% by mass, more preferably not less than 10% by mass and even more preferably not less than 20% by mass, and is also preferably not more than 100% by mass, from the viewpoint of finely controlling an external shape of the skin without damage to natural appearance thereof.

The vinyl-based polymer having the aforementioned siloxane dendrimer structure in a side chain thereof is preferably an acrylic polymer. That is, the preferred silicone dendrimer is an acrylic polymer having the siloxane dendrimer structure in a side chain thereof (hereinafter also referred to as an "acrylic silicone dendrimer"). The acrylic silicone dendrimer is a polymer containing a repeating unit derived from a monomer represented by the general formula (7) in which Y is a (meth)acryloyl group, and may further contain a repeating unit derived from a (meth)acrylic monomer other than the monomer represented by the general formula (7).

Examples of commercially available products of the silicone dendrimer include acrylic silicone dendrimers, such as "FA 4001 CM Silicone Acrylate" (a cyclopentasiloxane solution of an acrylate-polytrimethylsiloxymethacrylate copolymer) and "FA 4002 ID Silicone Acrylate" (an isododecane solution of an acrylate-polytrimethylsiloxymethacrylate copolymer) both available from Dow Corning Toray Co., Ltd., and the like.

The aforementioned silicone polymer is preferably at least one polymer selected from the group consisting of a silicone-modified polynorbornene, a silicone-modified pullulan, trimethylsiloxysilicic acid, trifluoropropyldimethyl/trimethylsiloxysilicic acid, and an acrylic silicone dendrimer, and more preferably at least one polymer selected from the group consisting of a silicone-modified polynorbornene, a silicone-modified pullulan, trimethylsiloxysilicic acid and trifluoropropyldimethyl/trimethylsiloxysilicic acid, from the viewpoint of finely controlling an external shape of the skin without damage to natural appearance thereof.

As described above, from the viewpoint of finely controlling an external shape of the skin without damage to natural appearance thereof, the polymer A is at least one polymer selected from the group consisting of a silicone polymer having the structure represented by the aforementioned general formula (I) and a vinyl-based polymer containing a repeating unit derived from an anionic group-containing vinyl-based monomer; more preferably a silicone-modified polymer, and a vinyl-based polymer containing a repeating unit derived from at least one vinyl-based monomer selected from the group consisting of acrylic acid, methacrylic acid, maleic acid, styrenesulfonic acid, and salts of these acids; even more preferably at least one polymer selected from the group consisting of a silicone-modified polynorbornene, a silicone-modified pullulan, trimethylsiloxysilicic acid, trifluoropropyldimethyl/trimethylsiloxysilicic acid, an acrylic silicone dendrimer, polymethacrylic acid, and sodium polystyrene sulfonate; and further even more preferably at least one polymer selected from the group consisting of a silicone-modified polynorbornene, a silicone-modified pullulan, trimethylsiloxysilicic acid, trifluoropropyldimethyl/trimethylsiloxysilicic acid, polymethacrylic acid, and sodium polystyrene sulfonate.

In addition, in the present invention, the effect of finely controlling an external shape of the skin is preferably well continuously maintained even when the coating film formed on the skin in the step 2 undergoes an external force or a physical deformation, etc. From this viewpoint, the polymer (A) is preferably the silicone polymer having the structure represented by the aforementioned general formula (I); more preferably the silicone-modified polymer; even more preferably at least one polymer selected from the group consisting of a silicone-modified polynorbornene, a silicone-modified pullulan, trimethylsiloxysilicic acid, trifluoropropyldimethyl/trimethylsiloxysilicic acid, and an acrylic silicone dendrimer; further even more preferably at least one polymer selected from the group consisting of a silicone-modified polynorbornene, a silicone-modified pullulan, trimethylsiloxysilicic acid, and trifluoropropyldimethyl/trimethylsiloxysilicic acid; still further even more preferably at least one polymer selected from the group consisting of a silicone-modified polynorbornene, a silicone-modified pullulan, and trifluoropropyldimethyl/trimethylsiloxysilicic acid; yet still further even more preferably at least one polymer selected from the group consisting of a silicone-modified polynorbornene, and a silicone-modified pullulan; furthermore preferably a silicone-modified polynorbornene; and yet furthermore preferably the silicone-modified polynorbornene represented by the aforementioned formula (4).

In the present invention, even though the content of the polymer A in the aforementioned liquid composition is low, and the liquid composition is in the form of a composition having a comparatively low concentration of the polymer A, it is possible to exhibit a contraction action of the skin without damage to natural appearance thereof by applying the fine liquid droplets of the liquid composition to the skin. In addition, in the case where the fine liquid droplets are applied to the skin by the below-mentioned ink-jet method or nano-dispenser method, by adjusting the formulation of the liquid composition so as to design a low content of the polymer A in the liquid composition, the viscosity of the liquid composition can be reduced, and the ejection properties of the liquid composition can be improved, so that it is possible to conduct pattern printing with a high accuracy. From this viewpoint, the content of the polymer A in the liquid composition is preferably not less than 0.1% by mass, more preferably not less than 0.5% by mass, even more preferably not less than 1% by mass, further even more preferably not less than 5% by mass, still further even more preferably not less than 7% by mass and yet still further even more preferably not less than 10% by mass, and is also preferably not more than 30% by mass, more preferably not more than 25% by mass, even more preferably not more than 20% by mass and further even more preferably not more than 15% by mass.

### (Volatile Solvent B)

The liquid composition used in the present invention contains the volatile solvent B from the viewpoint of finely controlling an external shape of the skin without damage to natural appearance thereof.

The volatile solvent B preferably has a volatilization rate of not less than 5% as measured after volatilizing the solvent under a pressure of 1 atm at a temperature of 40 °C and a humidity of 60% RH for 30 minutes. By controlling the volatilization rate of the volatile solvent B to the aforementioned range, it is possible to improve a drying velocity of the fine liquid droplets of the liquid composition in the step 2 and accelerate contraction of a coating film of the respective fine liquid droplets of the liquid composition, so that the effect of finely controlling an external shape of the skin without damage to natural appearance thereof can be enhanced.

From the same viewpoint as described above, the volatilization rate of the volatile solvent B is more preferably not less than 14%, even more preferably not less than 18%, further even more preferably not less than 25%, still further even more preferably not less than 30%, yet still further even more preferably not less than 45%, furthermore preferably not less than 55%, furthermore preferably not less than 60%, furthermore preferably not less than 65%, furthermore preferably not less than 70%, furthermore preferably not less than 80% and yet furthermore preferably not less than 95%, and the upper limit of the volatilization rate of the volatile solvent B is 100%.

The volatilization rate may be determined by placing 0.5 g of a sample to be measured in a glass petri dish, and allowing the sample to stand under environmental conditions of a pressure of 1 atm and a humidity of 60% RH at a temperature of 40 °C for 30 minutes to volatilize the solvent from the sample, and is calculated according to the formula: [(W₀ -W₁)/W₀] x 100 wherein W₀ is a mass (g) of the sample before volatilizing the solvent therefrom; and W₁ is a mass (g) of the sample after volatilizing the solvent therefrom. The larger the value of the volatilization rate becomes, the larger the volatilization velocity of the solvent is.

The volatilization rate can be more specifically measured by the method described in Examples below.

The volatile solvent B may be used alone or in combination of any two or more kinds thereof.

As the volatile solvent B, either a water-soluble solvent or a water-insoluble solvent may be appropriately selected and used according to the kind of polymer A. The term "water-soluble" as used herein means that a solubility of the solvent in water is more than 1 w/w%, whereas the term "water-insoluble" as used herein means that a solubility of the solvent in water is not more than 1 w/w%.

The volatile solvent B is preferably at least one solvent selected from the group consisting of water, an aliphatic alcohol having not less than 2 and not more than 4 carbon atoms, a volatile hydrocarbon oil and a volatile silicone oil, and more preferably at least one solvent selected from the group consisting of water (volatilization rate: 100%), ethanol (volatilization rate: 100%), isododecane (volatilization rate: 33%), hexamethyl disiloxane (volatilization rate: 100%), methyl trimethicone, and dimethyl polysiloxane having a dynamic viscosity of less than 5 mm²/s as measured at 25 °C.

The dynamic viscosity at 25 °C may be measured using an Ubbelohde viscometer according to ASTM D 445-46T or JIS Z 8803.

In the case where the polymer A is the silicone polymer having the structure represented by the aforementioned general formula (I), the volatile solvent B is preferably a water-insoluble solvent, more preferably at least one solvent selected from the group consisting of a volatile hydrocarbon oil and a volatile silicone oil, and even more preferably at least one solvent selected from the group consisting of isododecane (volatilization rate: 33%), hexamethyl disiloxane (volatilization rate: 100%), methyl trimethicone, and dimethyl polysiloxane having a dynamic viscosity of less than 5 mm²/s as measured at 25 °C.

In the case where the polymer A is the vinyl-based polymer containing the repeating unit derived from the anionic group-containing vinyl-based monomer, the volatile solvent B is preferably a water-soluble solvent, and more preferably at least one solvent selected from the group consisting of water, and an aliphatic alcohol having not less than 2 and not more than 4 carbon atoms.

Examples of commercially available products of the hexamethyl disiloxane and the dimethyl polysiloxane having a dynamic viscosity of less than 5 mm²/s as measured at 25 °C include "KF-96L-0.65cs" (hexamethyl disiloxane; dynamic viscosity: 1.0 mm²/s), "TMF1.5", "KF-96L-1cs" (octamethyl trisiloxane; dynamic viscosity: 1.0 mm²/s), "KF-96L-1.5cs" (decamethyl tetrasiloxane; dynamic viscosity: 1.5 mm²/s), "KF-96L-2cs" (dodecamethyl pentasiloxane; dynamic viscosity: 2.0 mm²/s), and "KF-995" (decamethyl cyclopentasiloxane; dynamic viscosity: 4.0 mm²/s) all available from Shin-Etsu Chemical Co., Ltd.; "SH200C Fluid 1cs" and "SH200C Fluid 1.5cs" both available from Dow Corning Toray Co., Ltd.; "TSF451-065" available from Momentive Performance Materials Japan Inc.; "BELSIL DM0.65" available from Wacker Asahikasei Silicone Co., Ltd.; and the like.

The content of the component (B) in the aforementioned liquid composition is preferably not less than 1% by mass, more preferably not less than 5% by mass, even more preferably not less than 10% by mass, further even more preferably not less than 20% by mass, still further even more preferably not less than 25% by mass, yet still further even more preferably not less than 35% by mass, furthermore preferably 45% by mass, furthermore preferably 50% by mass, furthermore preferably 60% by mass, furthermore preferably 70% by mass and yet furthermore preferably 80 % by mass from the viewpoint of finely controlling an external shape of the skin without damage to natural appearance thereof, and is also preferably not more than 99.9% by mass, more preferably not more than 99.5% by mass, even more preferably not more than 99% by mass, further even more preferably not more than 97% by mass and still further even more preferably not more than 95% by mass from the viewpoint of improving a degree of freedom of formulation of the liquid composition.

The value obtained by dividing a product of the volatilization rate (%) of the volatile solvent B and the content (% by mass) of the volatile solvent B in the liquid composition by 100 [[volatilization rate (%) x content (% by mass)]/100] is preferably not less than 5, more preferably not less than 20, even more preferably not less than 35, further even more preferably not less than 40, still further even more preferably not less than 45, yet still further even more preferably not less than 60 and furthermore preferably not less than 70, and is also preferably not more than 99.9, from the viewpoint of finely controlling an external shape of the skin without damage to natural appearance thereof.

The mass ratio of the content of the polymer A to the content of the volatile solvent B in the liquid composition (A/B) (hereinafter also referred to as a "content mass ratio (A/B)") is preferably not less than 0.05, more preferably not less than 0.07 and even more preferably not less than 0.1 from the viewpoint of finely controlling an external shape of the skin without damage to natural appearance thereof, and is also preferably not more than 0.3, more preferably not more than 0.25 and even more preferably not more than 0.2 from the viewpoint of well controlling a viscosity of the liquid composition to improve capability of applying the fine liquid droplets of the liquid composition.

### (Other Components)

The liquid composition used in the present invention may also contain the other components that may be usually formulated in cosmetics, etc., in addition to the polymer A and the volatile solvent B, unless the advantageous effects of the present invention are adversely affected by inclusion thereof. Examples of the other components include materials other than the polymer A and the volatile solvent B, such as an oil agent, a surfactant, a water-soluble polymer, an oxidation inhibitor, an ultraviolet absorber, an ultraviolet scattering agent, a vitamin preparation, an antiseptic agent, a pH modifier, a perfume, a beauty care ingredient, a medical ingredient, vegetable essences, a humectant, a colorant, a cooling agent, an antiperspirant, a germicide, an antiseptic agent, a skin activator, and the like. These other components may be used alone or in combination of any two or more thereof.

### [Non-Volatile Oil]

The liquid composition used in the present invention may further contain a non-volatile oil as an oil agent other than the volatile solvent B. The non-volatile oil means an oil having a volatilization rate of less than 5%. Examples of the non-volatile oil include those oils that are allowed to be present in a liquid state as measured under 1 atm at 25 °C, such as an ester oil, a silicone oil, a hydrocarbon oil, a higher fatty acid, a higher alcohol, and the like.

Examples of the liquid non-volatile oil include monoester oils, such as isotridecyl isononanoate, etc.; diester oils, such as polyglyceryl diisostearate, di(cholesteryl/behenyl/octyldodecyl) lauroyl glutamate, di(octyldodecyl/phytosteryl/behenyl) lauroyl glutamate, etc.; ester oils, such as glyceryl tri(caprylate/caprinate/myristate/stearate), etc.; silicone oils, such as dimethyl polysiloxane having a dynamic viscosity of not less than 5 mm²/s as measured at 25 °C, etc.; hydrocarbon oils, such as liquid paraffin, light liquid isoparaffin such as hydrogenated polyisobutene, etc., heavy liquid isoparaffin, liquid ozokerite, squalane, pristane, squalene, isohexadecane, etc.; higher fatty acids having not less than 12 and not more than 22 carbon atoms; higher alcohols having not less than 12 and not more than 28 carbon atoms; and the like.

The liquid composition may also contain a non-volatile oil that is allowed to be present in a solid state as measured under 1 atm at 25 °C, unless the advantageous effects of the present invention are adversely affected by inclusion thereof.

Examples of the solid non-volatile oil include solid ester oils, such as glyceryl monostearate, glyceryl monomyristate, etc.; solid silicone oils, such as alkyl-modified silicones, polyether-modified silicones, etc.; solid hydrocarbon oils, such as vaseline, etc.; solid higher alcohols, such as cetyl alcohol, stearyl alcohol, behenyl alcohol, etc.; solid higher fatty acids, such as lauric acid, myristic acid, palmitic acid, stearic acid, behenic acid, 12-hydroxystearic acid, lanolin fatty acids, etc.; and the like.

In the case where the liquid composition used in the present invention contains the non-volatile oil, the content of the non-volatile oil in the liquid composition is preferably not less than 0.3% by mass, more preferably not less than 0.5% by mass and even more preferably not less than 0.7% by mass from the viewpoint of improving capability of applying the fine liquid droplets to the skin, and is also preferably not more than 10% by mass, more preferably not more than 7% by mass, even more preferably not more than 5% by mass and further even more preferably not more than 3% by mass from the viewpoint of finely controlling an external shape of the skin without damage to natural appearance thereof as well as from the viewpoint of suppressing stickiness of the skin when applying the composition to the skin.

### [Crosslinked-Type Silicone Resin]

The liquid composition used in the present invention may further contain a crosslinked-type silicone resin unless the advantageous effects of the present invention are adversely affected by inclusion thereof.

Examples of the crosslinked-type silicone resin include those crosslinked-type silicone resins obtained by crosslinking silicone resins with divinyl dimethyl polysiloxane (vinyl dimethicone), phenylvinyl dimethyl polysiloxane (phenylvinyl dimethicone), PEG-10 diallyl ether, PEG-15 diallyl ether, and the like. Specific examples of the crosslinked-type silicone resin include a (dimethicone/vinyl dimethicone) crosspolymer, a (vinyl dimethicone/methicone silsesquioxane) crosspolymer, a (vinyl dimethicone/lauryl dimethicone) crosspolymer, a (dimethicone/(PEG-10/15)) crosspolymer, a (PEG-15/lauryl dimethicone) crosspolymer, a (dimethicone/phenylvinyl dimethicone) crosspolymer, and the like.

Examples of commercially available products of the crosslinked-type silicone resin include "KSP" series products, "KMP" series products and "KSG" series products all available from Shin-Etsu Chemical Co., Ltd., and the like.

In the case where the liquid composition used in the present invention contains the crosslinked-type silicone resin, the content of the crosslinked-type silicone resin in the liquid composition is preferably not less than 0.2% by mass, more preferably not less than 0.5% by mass and even more preferably not less than 0.7% by mass from the viewpoint of improving capability of applying the fine liquid droplets to the skin, and is also preferably not more than 10% by mass, more preferably not more than 7% by mass and even more preferably not more than 5% by mass from the viewpoint of finely controlling an external shape of the skin without damage to natural appearance thereof.

In the case where the liquid composition contains the component(s) other than the polymer A and the volatile solvent B, the total content of the polymer A and the volatile solvent B in the liquid composition is preferably not less than 80% by mass, more preferably not less than 85% by mass, even more preferably not less than 90% by mass and further even more preferably not less than 95% by mass from the viewpoint of finely controlling an external shape of the skin without damage to natural appearance thereof. In addition, the upper limit of the total content of the polymer A and the volatile solvent B in the liquid composition is 100% by mass.

In the case where the liquid composition contains the component(s) other than the polymer A and the volatile solvent B, the mass ratio of the content of the polymer A to the content of the other component(s) in the liquid composition (hereinafter also referred to as a "content mass ratio (A/other component(s))") is preferably not less than 1, more preferably not less than 2 and even more preferably not less than 3, and is also preferably not more than 20, more preferably not more than 17 and even more preferably not more than 15, from the viewpoint of finely controlling an external shape of the skin without damage to natural appearance thereof.

The viscosity of the liquid composition as measured at 35 °C is preferably not less than 1 mPa•s, more preferably not less than 1.5 mPa•s and even more preferably not less than 2 mPa•s from the viewpoint of suppressing occurrence of shiny smooth feel over a whole surface of the skin treated, and improving a contraction action of the skin without damage to natural appearance thereof, and is also preferably not more than 20 mPa•s, more preferably not more than 15 mPa•s and even more preferably not more than 10 mPa•s from the viewpoint of conducting pattern printing with a high accuracy. The viscosity at 35 °C of the liquid composition may be measured using an E-type viscometer "TV-25" (equipped with a standard cone rotor (1°34' x R24); rotating speed: 50 rpm) available from Toki Sangyo Co., Ltd.

### <Application of Fine Liquid Droplets>

The method of applying the fine liquid droplets of the liquid composition to the skin in the step 1 is not particularly limited as long as the mass of the fine liquid droplets applied to a desired region of the skin as measured per one droplet thereof can be controlled to the predetermined range. Examples of the method of applying the fine liquid droplets of the liquid composition include the method of applying the fine liquid droplets of the liquid composition by pattern printing used in printing systems, the method of spraying the fine liquid droplets of the liquid composition onto the skin that is partially masked, using a spraying device (sprayer), etc., and the like. Among these methods, preferred is the method conducted by the pattern printing used in printing systems. By using the pattern printing, it is possible to well control the mass of the fine liquid droplets of the liquid composition applied in the step 1 as measured per one droplet thereof, the amount of the fine liquid droplets applied per a unit area, the region to which the fine liquid droplets are applied, and the like, and as a result, it is possible to well control contraction of the skin in the step 2 in a planar or three-dimensional manner. In addition, by properly designing a print pattern used in the pattern printing, it is possible to preliminarily design the external shape of the skin after being treated. Moreover, since the pattern printing if used is capable of enhancing reproducibility of the skin treatment, the skin treatment method of the present invention can also be adapted for non-invasive cosmetic surgery technologies or beauty care procedures in esthetic salon, etc. Among them, the skin treatment method of the present invention is preferably applied to a beauty care method in which the beauty care procedures are performed, from the viewpoint of improving non-invasiveness, reproducibility and convenience of the skin treatments.

Examples of the pattern printing used in the present invention include on-demand printing, such as printing by an ink-jet method, printing by a dispenser method, etc.; and analog printing, such as screen printing, flexographic printing, gravure printing, offset printing, etc. These printing methods may be selectively used according to the viscosity of the liquid composition, and the like.

The on-demand printing is a plateless printing method requiring no printing plate in which the printing is conducted in non-contact with the skin.

The analog printing is a plate-based printing method requiring a printing plate in which the printing is conducted in contact with the skin.

Among these printing methods, the on-demand printing is capable of ejecting the fine liquid droplets of the liquid composition to apply a desired amount of the fine liquid droplets to a desired region of the skin, and well controlling the mass of the fine liquid droplets applied as measured per one droplet thereof, the amount of the fine liquid droplets applied per an unit area, the region to which the fine liquid droplets are to be applied, and the like, so that it is possible to facilitate fine control of an external shape of the skin. From this viewpoint, as the method of applying the fine liquid droplets of the liquid composition to the skin in the step 1, preferred is the method in which the liquid composition is ejected by at least one method selected from the group consisting of an ink-jet method and a dispenser method to apply the fine liquid droplets thereof to the skin.

The mass of the fine liquid droplets applied in the step 1 as measured per one droplet thereof is not less than 0.001 µg, preferably not less than 0.005 µg, more preferably not less than 0.01 µg, even more preferably not less than 0.02 µg and further even more preferably not less than 0.03 µg, and is also not more than 100 µg, preferably not more than 50 µg, more preferably not more than 30 µg, even more preferably not more than 10 µg, further even more preferably not more than 7 µg, still further even more preferably not more than 5 µg and yet still further even more preferably not more than 3 µg, from the viewpoint of finely controlling an external shape of the skin without damage to natural appearance thereof.

In the case where the method of applying the fine liquid droplets is, for example, the method in which the liquid composition is ejected by an ink-jet method, a nano-dispenser method, etc., to apply the fine liquid droplets thereof to the skin, the mass of the fine liquid droplets applied as measured per one droplet thereof may be calculated from an average diameter of the fine liquid droplets ejected to the skin, or the change in mass of the liquid composition when ejecting a predetermined number of the fine liquid droplets.

In addition, in the case where the method of applying the fine liquid droplets is a plate-based printing method, the mass of the fine liquid droplets applied as measured per one droplet thereof may be calculated from a size of a hole or a cell formed in a printing plate, such as a stencil, an intaglio, etc.

The amount of the fine liquid droplets applied per an unit area in the step 1 in terms of the amount of the polymer A applied is preferably not less than 0.1 mg/cm², more preferably not less than 0.2 mg/cm², even more preferably not less than 0.3 mg/cm², further even more preferably not less than 0.5 mg/cm² and still further even more preferably not less than 0.7 mg/cm², and is also preferably not more than 3 mg/cm², more preferably not more than 2 mg/cm² and even more preferably not more than 1.5 mg/cm², from the viewpoint of finely controlling an external shape of the skin without damage to natural appearance thereof.

The interval between the fine liquid droplets applied to the skin in the step 1 (a center distance between the adjacent fine liquid droplets) is preferably not less than 0.01 mm, more preferably not less than 0.03 mm, even more preferably not less than 0.05 mm, further even more preferably not less than 0.1 mm, still further even more preferably not less than 0.3 mm and yet still further even more preferably not less than 0.5 mm, and is also preferably not more than 2 mm, more preferably not more than 1.5 mm and even more preferably not more than 1 mm.

Incidentally, in the case where the fine liquid droplets of the liquid composition are applied to the skin in the step 1 by a pattern printing method, it is possible to suitably control the interval between the fine liquid droplets applied, by adjusting a print pitch in the pattern printing.

The average diameter of the fine liquid droplets applied to the skin in the step 1 is preferably not less than 10 µm, more preferably not less than 20 µm and even more preferably not less than 30 µm, and is also preferably not more than 500 µm, more preferably not more than 450 µm, even more preferably not more than 400 µm and further even more preferably not more than 350 µm.

In the case where the fine liquid droplets of the liquid composition are applied to the skin in the step 1 by a pattern printing method used in printing systems, the ratio of the interval between the fine liquid droplets (print pitch) to the average diameter of the fine liquid droplets applied in the step 1 (print pitch/average diameter of fine liquid droplets) is preferably not less than 0.3, more preferably not less than 0.7 and even more preferably not less than 1, and is also preferably not more than 3, more preferably not more than 2.5 and even more preferably not more than 2, from the viewpoint of finely controlling an external shape of the skin without damage to natural appearance thereof, in particular, from the viewpoint of attaining a good wrinkle improving effect.

In addition, the ratio of the interval between the fine liquid droplets (print pitch) to the average diameter of the fine liquid droplets applied in the step 1 (print pitch/average diameter of fine liquid droplets) is preferably not less than 0.3, more preferably not less than 0.7 and even more preferably not less than 1, and is also preferably not more than 3, more preferably not more than 2.5, even more preferably not more than 2 and further even more preferably not more than 1.5, from the viewpoint of improving the effect of forming undulation on the skin.

The interval between the fine liquid droplets and the average diameter of the fine liquid droplets applied to the skin may be measured by observation using an optical microscope in which 20 objects are measured for these items to obtain an average value thereof.

In the case where the method of applying the fine liquid droplets in the step 1 is conducted by an ink-jet method, as the ink-jet ejecting method, there may be used either a thermal method or a piezoelectric method.

The voltage applied to a ink-jet ejection head is preferably not less than 5 V, more preferably not less than 10 V and even more preferably not less than 15 V, and is also preferably not more than 50 V, more preferably not more than 45 V and even more preferably not more than 40 V, from the viewpoint of finely controlling an external shape of the skin without damage to natural appearance thereof.

The drive frequency of the ink-jet ejection head is preferably not less than 1 kHz and more preferably not less than 3 kHz, and is also preferably not more than 300 kHz, more preferably not more than 150 kHz, even more preferably not more than 90 kHz and further even more preferably not more than 50 kHz, from the viewpoint of finely controlling an external shape of the skin without damage to natural appearance thereof.

In the case where the method of applying the fine liquid droplets in the step 1 is a nano-dispenser method, as the nano-dispenser ejecting method, preferred is a jet method capable of ejecting the liquid composition in non-contact with a substrate to be printed, and more preferred is a jet method using a piezoelectric element.

The voltage applied to a nano-dispenser ejection head is preferably not less than 5 V, more preferably not less than 10 V and even more preferably not less than 15 V, and is also preferably not more than 100 V, more preferably not more than 80 V and even more preferably not more than 60 V, from the viewpoint of finely controlling an external shape of the skin without damage to natural appearance thereof.

The drive frequency of the nano-dispenser ejection head is preferably not less than 1 Hz and more preferably not less than 5 Hz, and is also preferably not more than 30 kHz, more preferably not more than 10 kHz, even more preferably not more than 5 kHz and further even more preferably not more than 1 kHz, from the viewpoint of finely controlling an external shape of the skin without damage to natural appearance thereof.

In the step 1, the fine liquid droplets of the liquid composition may be uniformly applied over a whole surface of the region of the skin to which it is desired to apply the fine liquid droplets. However, from the viewpoint of finely controlling an external shape of the skin without damage to natural appearance thereof, by suitably adjusting the print pattern, the number of printing operations, the order of application of the fine liquid droplets, and the like, to control contraction of the skin, it is possible to facilitate control of an external shape of the skin.

The print pattern used in the pattern printing in the step 1 may be designed in view of contraction of the skin according to the external shape of the skin after being treated. As the print pattern, there may be used, for example, those print patterns having a triangular grid shape, a square grid shape, a rectangular grid shape and a honeycomb grid shape.

In addition, there may also be used such a print pattern as prepared according to shapes of respective skin hills on the skin to be treated. For example, when applying the fine liquid droplets to the respective skin hills on the skin, the skin undergoes contraction in association with drying of the fine liquid droplets applied to the respective skin hills, so that it is possible to form respective skin hills each having a raised-up shape and thereby increase the difference between a convex shape of the respective skin hills and a concave shape of respective skin grooves. As a result, clearness of the skin texture can be enhanced, so that it is possible to suitably improve appearance of the skin.

In the case where the fine liquid droplets are applied in the step 1 by a pattern printing method, as the pattern printing method, there may be used either a printing method in which a desired amount of the fine liquid droplets are applied to a whole area of a desired region of the skin by only one printing operation, or a printing method in which divided parts of a desired amount of the fine liquid droplets are selectively applied to respective divided areas of a desired region of the skin by a plurality of printing operations. Among these printing methods, from the viewpoint of finely controlling an external shape of the skin without damage to natural appearance thereof, preferred is the printing method in which divided parts of a desired amount of the fine liquid droplets are selectively applied to respective divided areas of a desired region of the skin by a plurality of printing operations. By such a printing method as conducted by a plurality of printing operations, it is possible to well control the amount of the liquid composition applied to each region of the skin to be treated therewith, so that a contraction action of the skin can be suitably controlled.

Incidentally, in the case where the pattern printing is conducted by a plurality of printing operations, it is preferred that the step 2 is conducted after completion of every printing operation. By conducting the step 2 after completion of every pattern printing operation, the fine liquid droplets applied in the respective printing operations can be dried each time, and prevented from being mixed with each other, whereby it is possible to conduct a finely controlled skin treatment.

Even in the case where the fine liquid droplets are uniformly applied over a whole surface area of the region of the skin to be treated, by suitably adjusting the region of the skin to which the fine liquid droplets are to be applied, and the order of application of the fine liquid droplets thereto, it is possible to conduct the finely controlled skin treatment.

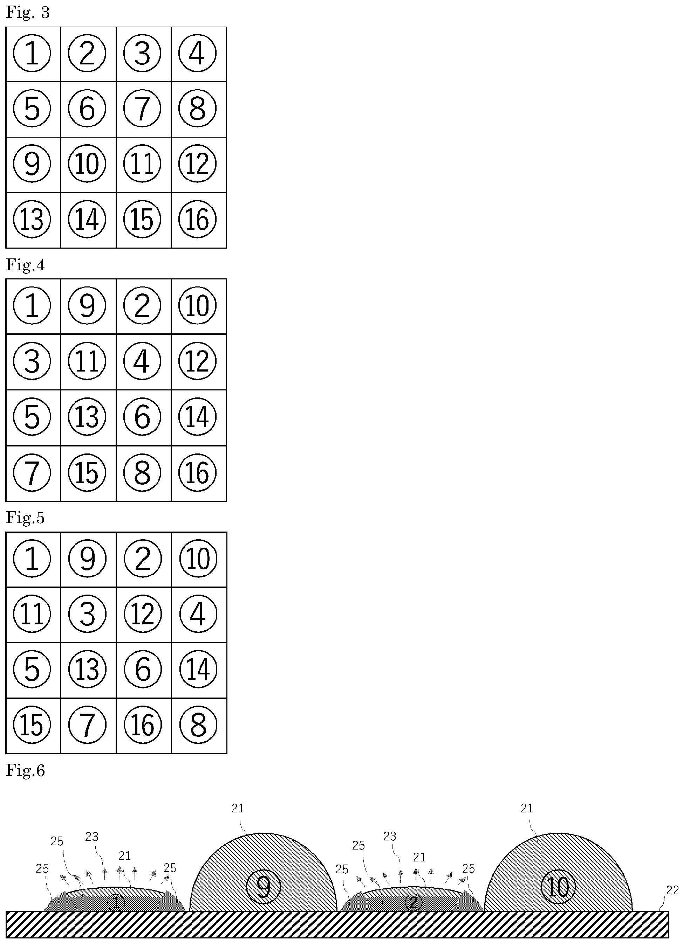

For example, in the case where the print pattern is of a square grid shape, as shown in FIG. 3, there may be used a method (1) in which the fine liquid droplets are sequentially applied to respective grids of the print pattern of the square grid shape in ascending order of the numbers shown in FIG. 3, or as shown in FIG. 4 and FIG. 5, there may be used a method (2) in which the fine liquid droplets are first alternately applied to the grids of the print pattern of the square grid shape in ascending order of the numbers shown in FIG. 4 and FIG. 5 while skipping every other grid, and then sequentially applied to the grids skipped in the previous operation in ascending order of the numbers shown in FIG. 4 and FIG. 5. Among these methods, from the viewpoint of finely controlling an external shape of the skin without damage to natural appearance thereof, preferred is the method (2). By conducting the method (2), it is possible to further enhance the contraction action of the skin in proportion to the amount of the polymer A applied. The reason why the aforementioned advantageous effect can be attained is considered as follows. That is, by alternately applying the fine liquid droplets to the grids of the print pattern to be applied therewith while skipping every other grid, it is possible to reduce a vapor pressure of the volatile solvent B present in the periphery of the respective fine liquid droplets after being applied to the skin, and thereby accelerate drying of the fine liquid droplets. Moreover, as shown in the cross-sectional view of FIG. 6, the skin is fixed and contracted by the first fine liquid droplets alternately applied earlier (shown by the numbers 1 and 2 in FIG. 6). Therefore, it is considered that by subsequently alternately applying the second fine liquid droplets to the respective grid regions skipped in the previous operation (shown by the numbers 9 and 10 in FIG. 6), the skin fixed by the first fine liquid droplets applied thereto undergoes a further contraction action.

In the present invention, by suitably adjusting not only the mass of the fine liquid droplets applied as measured per one droplet thereof and the amount of the fine liquid droplets applied as measured per a unit area, but also the number and density of the fine liquid droplets applied in the direction perpendicular to the contraction direction of the skin as desired, the order of application of the fine liquid droplets, and drying conditions of the fine liquid droplets, it is possible to well control the contraction direction of the skin and the amount of contraction of the skin.

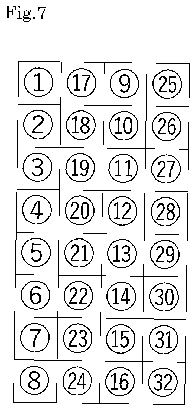

For example, when using the print pattern of a rectangular grid shape as shown in FIG. 7, in the step 1, the first fine liquid droplets can be continuously applied along a vertically long distance in ascending order of the numbers 1 to 8 as shown in FIG. 7 and then further applied along an alternately arranged row of the grids in ascending order of the numbers 9 to 16 as shown in FIG. 7 while skipping every other row of the grids arranged therebetween. By conducting such a procedure, the fine liquid droplets with the numbers of 1 to 16 are continuously applied in the vertical direction, so that the contraction of the skin owing to volatilization of the volatile solvent B therefrom becomes similar to the case where the fine liquid droplets are applied over a whole surface area of the skin. On the other hand, since a gap is formed by one row of the skipped grids in the lateral direction of the print pattern, the volatile solvent B tends to be easily volatilized from the fine liquid droplets, so that the degree of contraction of the fine liquid droplets with the numbers of 1 to 16 becomes almost similar to the case where the respective fine liquid droplets are applied singly. Next, the fine liquid droplets with the numbers of 17 to 32 are applied to vacant gaps as the respective rows of the grids skipped in the previous operation. Since a large portion of the volatile solvent B contained in the fine liquid droplets with the numbers of 1 to 16 have been already volatilized, and scattered or diffused, the volatile solvent B contained in the fine liquid droplets with the numbers of 17 to 32 can be hardly inhibited from being volatilized, so that the degree of contraction of the skin in the lateral direction becomes almost similar to the case where the respective fine liquid droplets are applied singly. As a result, the contraction action of the skin in the vertical direction becomes similar to the case where the fine liquid droplets are uniformly applied over a whole surface area of the skin, whereas the contraction action of the skin in the lateral direction is strengthened as compared to the case where the fine liquid droplets are uniformly applied over a whole surface area of the skin.

Thus, by using the skin treatment method of the present invention, the degree of contraction action of the skin and the degree of freedom of controlling the skin contraction in the respective directions can be enhanced.

In the skin treatment method of the present invention, by suitably selecting parts of human body to which the fine liquid droplets are to be applied, it is possible to finely control an external shape of the skin without damage to natural appearance thereof. Among them, the skin treatment method of the present invention is preferably applied to a makeup method that is used as a method of improving wrinkles on the skin or a method of forming undulation on the skin.

In the case where the skin treatment method of the present invention is used as the method of improving wrinkles on the skin, the fine liquid droplets of the liquid composition are preferably applied to peripheral portions of the wrinkles on the skin. By applying the fine liquid droplets to the peripheral portions of the wrinkles, the skin present in the peripheral portions of the wrinkles is contracted in association with drying of the fine liquid droplets in the subsequent step 2, so that recessed portions on the skin which form the wrinkles are flattened to make the wrinkles inconspicuous. In this case, by suitably adjusting the mass of the fine liquid droplets applied as measured per one droplet thereof, the amount of the liquid composition applied, etc., according to a size or a depth of the respective wrinkles in the step 1, it is possible to use the skin treatment method of the present invention without any particular limitation to the kinds of wrinkles or parts of human body. Among them, the skin treatment method of the present invention is preferably used as the method of improving fine wrinkles being present in an outer corner of the eye.

The "peripheral portions of the wrinkles on the skin" as used in the present specification are distinguished from inside portions of the wrinkles, i.e., concave portions on the surface of the skin or concave portions in streaks on the surface of the skin. Incidentally, in the case where the skin treatment method of the present invention is used as the method of improving wrinkles on the skin, the fine liquid droplets of the liquid composition can be applied to not only the peripheral portions of the wrinkles on the skin, but also the inside portions of the wrinkles as long as the advantageous effects of the present invention can be attained thereby.

From the viewpoint of attaining the effect of making the wrinkles inconspicuous, among the peripheral portions of the wrinkles, the region to which the fine liquid droplets of the liquid composition are to be applied preferably includes at least a part of a region extending in the direction that intersects with the wrinkle-extending direction. The wrinkle-extending direction means the direction in which streaks of the wrinkles run. This is because by applying the fine liquid droplets of the liquid composition to the region extending in the direction that intersects with the wrinkle-extending direction, the skin in the portion where the wrinkles are formed is stretched so that the wrinkles are made inconspicuous.

The region extending in the direction that intersects with the wrinkle-extending direction is preferably a region located along the wrinkle-extending direction, and may be present on either one side or both sides of the wrinkles. In particular, it is preferred that the fine liquid droplets of the liquid composition are applied to both sides of the wrinkles, i.e., such regions between which the wrinkles are sandwiched. This is because by applying the fine liquid droplets to both sides of the wrinkles, the effect of pulling the portion of the skin where the wrinkles are formed, from both sides thereof can be exerted, so that the wrinkles can be made inconspicuous.

In addition, in the case where the wrinkle-extending direction is not parallel to the direction of gravity, for example, as in nasolabial fold lines, it is preferred that the aforementioned region to which the liquid composition is to be applied (the region extending in the direction that intersects with the wrinkle-extending direction) includes a region located on an upper side of the wrinkles. The "direction of gravity" as used herein means the direction in which the gravity acts on a person in the case where the person stands upright, and the "upper side" means the direction opposite to the direction of gravity. By applying the aforementioned composition to at least the region on an upper side of the wrinkles, the portion of the skin where the wrinkles are formed is pulled upward, whereby it is possible to make the wrinkles more inconspicuous. From the same viewpoint as described above, in the case where the wrinkle-extending direction is not parallel to the direction of gravity, it is more preferred that the region to which the aforementioned liquid composition is to be applied includes those regions located on the upper and lower sides of the wrinkles between which the wrinkles are sandwiched, and the area of the region located on the upper side of the wrinkles is larger than the area of the region located on the lower side of the wrinkles.

In addition, in the case where the skin treatment method of the present invention is used as the method of forming undulation on the skin, the fine liquid droplets of the liquid composition are preferably applied in the step 1 to the region where it is desired to make unevenness on the surface of the skin conspicuous. By applying the fine liquid droplets to such a region, the skin present in the region where it is desired to make unevenness on the surface of the skin conspicuous is contracted in association with drying of the fine liquid droplets in the subsequent step 2, so that the skin tissue of the region is three-dimensionally lifted up to form undulation owing to the lifted skin, whereby the unevenness on the surface of the skin can be made conspicuous to impart a three-dimensional feel thereto.

In the case where the skin treatment method of the present invention is used as the method of forming undulation on the skin, by suitably adjusting the mass of the fine liquid droplets as measured per one droplet thereof, the amount of the liquid composition applied, etc., according to a size or a range of the undulation as desired, it is possible to apply the fine liquid droplets of the liquid composition to the skin without any particular limitation to parts of human body. Among them, the skin treatment method of the present invention is preferably used as the method of improving a shape of eye bags.

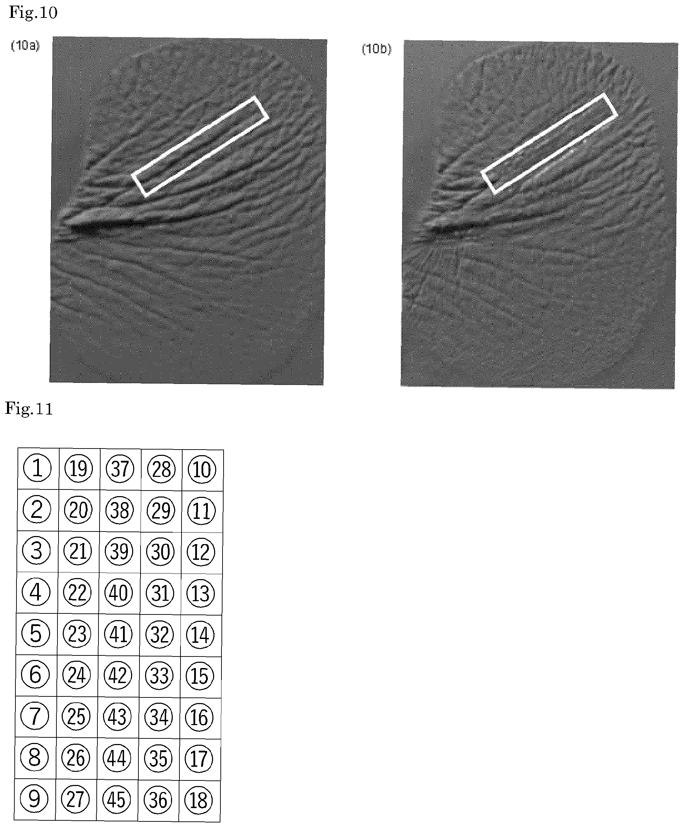

Referring to FIGS. 11 to 13, there are shown schematic views of print patterns having a rectangular grid shape which are used in the method of improving a shape of eye bags.

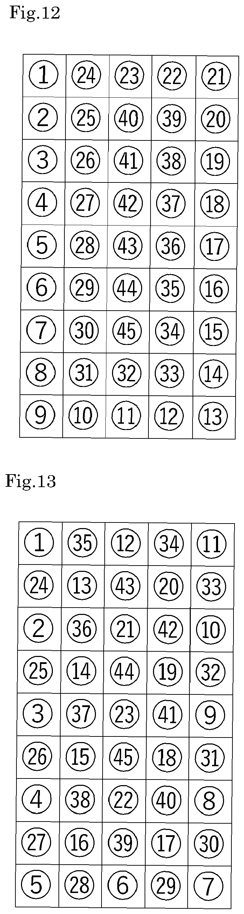

In the method of improving a shape of eye bags, as the method of applying the fine liquid droplets in the step 1, there may be mentioned, for example, the method (3) in which the fine liquid droplets are applied to the respective grids of the print pattern of a rectangular grid shape in ascending order of the numbers shown in FIG. 11 from the opposite end rows towards the central row of the grids of the print pattern as shown in FIG. 11, or the method (4) in which the fine liquid droplets are applied in a spiral manner to the respective grids of the print pattern of a rectangular grid shape in ascending order of the numbers shown in FIG. 12 from the outer periphery toward a center of the print pattern of the rectangular grid shape.

In addition, although the fine liquid droplets are applied in a spiral manner to the respective grids of the print pattern similarly to the aforementioned method (4), there may also be mentioned the method (5) in which as shown in FIG. 13, the fine liquid droplets are first alternately applied to the respective grids of the print pattern while skipping every other grid, and then alternately applied to the respective vacant grids skipped in the previous operation, in ascending order of the numbers as shown. Among these methods, from the viewpoint of finely controlling an external shape of the skin without damage to natural appearance thereof, preferred is the method (5). By conducting the method (5), it is possible to further lift up the skin in proportion to the amount of the polymer A applied. The reason why the aforementioned advantageous effects can be attained is considered as follows. That is, by alternately applying the fine liquid droplets while skipping every other grid, it is possible to reduce a vapor pressure of the volatile solvent B present in the periphery of the respective fine liquid droplets after being applied to the skin, and thereby accelerate drying of the fine liquid droplets. Moreover, as shown in FIG. 13, the skin is fixed and contracted by the first fine liquid droplets alternately applied earlier. Therefore, it is considered that by lately alternately applying the second fine liquid droplets to the respective regions skipped in the previous operation, the skin fixed by the first fine liquid droplets applied thereto undergoes a further contraction action.

Incidentally, in the print patterns shown in FIGS. 11 to 13, for the sake of convenience, there are illustrated those print patterns in the case where the number of the fine liquid droplets applied is 45. However, print patterns actually used are much finer, and formed of a much larger number of the fine liquid droplets applied thereto. As described above, by applying the fine liquid droplets to the region that is to be treated therewith in the step 1 from an outside toward an inside of the region, it is possible to lift up a central portion of the region to which the fine liquid droplets are applied, and thereby form an undulation shape that is lifted up like a shape of eye bags.

### (Step 2)

The step 2 is the step of drying the fine liquid droplets applied to the skin in the step 1 to contract the skin in association with the drying.

In the present invention, since the fine liquid droplets whose mass lies within the predetermined range as measured per one droplet thereof are applied in the step 1, the drying conditions of the fine liquid droplets in the step 2 are not particularly limited. The fine liquid droplets can be dried to a sufficient extent by natural drying at the temperature of the skin. In addition, from the viewpoint of accelerating the drying, the fine liquid droplets may be dried by blow drying, hot air drying, and the like.

In the case where the hot air drying is used for drying the fine liquid droplets, the temperature used upon the hot air drying is not particularly limited. The drying temperature is preferably not lower than 35 °C, more preferably not lower than 40 °C, even more preferably not lower than 50 °C and further even more preferably not lower than 55 °C, and is also preferably not higher than 80 °C, more preferably not higher than 70 °C and even more preferably not higher than 65 °C.

The drying time is preferably not less than 5 minutes, more preferably not less than 7 minutes and even more preferably not less than 10 minutes, and is also preferably not more than 30 minutes and more preferably not more than 20 minutes.

After completion of the step 2, the coating film of the liquid composition formed on the skin in the step 2 may be removed if required, or makeup may be applied onto the coating film formed on the skin in the step 2 without removing the coating film.

In the present invention, owing to contraction of the skin in association with the drying of the fine liquid droplets in the step 2, a papillary layer under the skin is deformed, and once deformed, the papillary layer thus deformed is slowly returned to its original shape, so that even after removing the coating film formed on the skin in the step 2, the advantageous effect owing to the aforementioned mechanism of occurrence of the skin contraction can be also attained. From this viewpoint, makeup can also be applied by applying the coating film formed on the skin in the step 2 for a predetermined period of time and removing the coating film from the skin immediately before applying makeup to the skin.

### EXAMPLES

The present invention is described below by referring to the following Examples, etc., but it should be construed that the present invention is not limited to these Examples, etc. In the following Examples, etc., various measurements were conducted by the following methods.

### (Number-Average Molecular Weight Mn and Weight-Average Molecular Weight Mw)

The number-average molecular weight Mn and the weight-average molecular weight Mw of the polymer were measured under the following conditions by gel permeation chromatography (GPC) using polystyrenes as a reference standard substance.
Measuring apparatus: "HLC-8320GPC" available from Tosoh Corporation;
Columns: "K-806L" available from Shodex, two columns connected in series;
Detector: RI;
Eluent: 1 mmol/L-FARMIN DM20 available from Kao Corporation/CHCl₃;
Flow rate: 1.0 mL/min; and
Column temperature: 40 °C.

### (Deformation Ratio)

The deformation ratio of the polymer A was determined by the following method as the method (1).

First, the polymer A was dissolved in the volatile solvent B to prepare a polymer solution having a concentration of 10% by mass. The polymer solution in an amount of 0.005 g was applied to one of surfaces of a polyethylene sheet 1 "LL Film" available from ENSHU CHEMICAL INDUSTRIES Inc., having a size of a width (W) of 20 mm, a length (L0) of 100 mm and a thickness of 0.03 mm to coat its region of a width (W) of 20 mm and a length (L1) of 50 mm extending from an end 1a of a short side of the polyethylene sheet 1 along a longitudinal direction thereof (the portion 2 shown in FIG. 1(a)) with the polymer solution, and then dried in a constant temperature oven set at 40 °C for 10 minutes to thereby prepare a polyethylene sheet 1' with a polymer film.

FIG. 1(b) is a schematic view of the polyethylene sheet 1' a part of which was deformed (curled) by contraction of the polymer film containing the polymer A when forming the polymer film by applying the polymer solution to the polyethylene sheet 1 and then drying the polymer solution.

When the length of the portion of the polyethylene sheet 1 onto which the polymer solution was applied was represented by L1 (50 mm), and the length of the portion of the polyethylene sheet 1' with the polymer film which underwent deformation (curling) by drying the polymer solution applied was represented by L2, the value of the ratio of L2 to L1 (L2/L1) was calculated and defined as the deformation ratio of the polymer A. Incidentally, L2 was determined as an average value of lengths measured on opposite two long sides of the polyethylene sheet 1' with the polymer film.

### (Volatilization Rate)

The volatilization rate (%) was determined as follows. That is, 0.5 g of a sample to be measured was placed in a glass petri dish having diameter of 40 mm, and allowed to stand on a hot plate set at a temperature of 40 °C for 30 minutes under environmental conditions of a pressure of 1 atm and a humidity of 60% RH to volatilize a solvent therefrom. The volatilization rate (%) was calculated according to the formula: [(W₀ -W₁)/W₀] x 100 wherein W₀ is a mass (g) of the sample before volatilizing the solvent therefrom; and W₁ is a mass (g) of the sample after volatilizing the solvent therefrom. The larger the value of the volatilization rate becomes, the larger the volatilization velocity of the solvent is.

### Preparation Example 1 (Preparation of Polymethacrylic Acid Solution)

A glass reaction container was charged with 300 g of methacrylic acid (guaranteed reagent) available from FUJIFILM Wako Pure Chemical Corporation, 1.5 L of ethanol (first-class grade reagent) available from FUJIFILM Wako Pure Chemical Corporation and 1.73 g of 2,2'-azobis(2,4-dimethyl valeronitrile) (first-class grade reagent) as a polymerization initiator available from FUJIFILM Wako Pure Chemical Corporation, and the contents of the reaction container were subjected to polymerization reaction at 65 °C for 4 hours. The thus obtained polymer solution was added dropwise to 20 L of acetone to subject the solution to reprecipitation. The resulting precipitate was recovered by filtration, and then dried under a reduced pressure of 10 kPa at 65 °C for 12 hours, thereby obtaining a polymethacrylic acid. The thus obtained polymethacrylic acid had a weight-average molecular weight of 170,000.

The resulting polymethacrylic acid was dissolved in ethanol (first-class grade reagent) available from FUJIFILM Wako Pure Chemical Corporation to prepare a polymethacrylic acid solution having a concentration of 10% by mass.

### improvement of Fine Wrinkles at Outer Corner of Eye>

### Examples 1 to 39

### (Step 1)

Using the respective liquid compositions and conditions shown in Tables 1 to 4, pattern printing was performed on an area having a longitudinal length of 5.6 mm and a lateral length of 10.4 mm on an artificial skin model "BIOSKIN, MODEL OF WRINKLES AT OUTER CORNER OF EYE" (product number : No. 145-2; wrinkle grade: 4) as a replica sample of skin available from Beaulax Co., Ltd., to apply fine liquid droplets of the respective liquid compositions thereto. Incidentally, the pattern printing was conducted on peripheral portions of the wrinkles on a wrinkle model formed on the replica sample while avoiding the portion of the replica sample where such deep wrinkles as generated at an outer corner of the eye were formed.

As a pattern printing apparatus, there were used a piezoelectric jet dispenser "PICO Pµlse Valve SD" available from Nordson EFD Corporation and a fluid body assembly "FLUID ASSY Pµlse MST 3.0S F0 E05" (opening: 50 µm; ball size: 3.0S). Incidentally, the dispenser was moved using "PRO4" available from Nordson Corporation.

Meanwhile, the mass of the fine liquid droplets as measured per one droplet thereof shown in Tables 1 to 4 was calculated from the change in mass of the liquid composition when ejecting the fine liquid droplets. The average diameter of the fine liquid droplets applied and the print pitch were measured by observation using an optical microscope in which 20 objects were measured as to each of these items to calculate an average value thereof as the measured value to be determined. The print patterns shown in Tables 1 to 4 are as follows.

Print pattern 1: As shown in FIG. 3, using a square grid pattern, the fine liquid droplets were applied to the respective grids in the pattern from an end thereof along the lateral direction in ascending order of the numbers shown in FIG. 3 to conduct the printing uniformly over a whole surface area of the region to be printed.

Print pattern 2: As shown in FIG. 4, using a square grid pattern, the fine liquid droplets were alternately applied to the respective grids in the pattern from an end thereof along the lateral direction in ascending order of the numbers shown in FIG. 4, and then the fine liquid droplets were also alternately applied to the non-treated grids in the pattern which were skipped in the previous operation, in ascending order of the numbers shown in FIG. 4. In this case, with respect to the grids arranged in the vertical direction of the pattern, the printing was sequentially conducted on the respective adjacent grids without skipping every other grid.

Print pattern 3: As shown in FIG. 5, using a square grid pattern, the fine liquid droplets were alternately applied to the respective grids in the pattern from an end thereof along the lateral and vertical directions in ascending order of the numbers shown in FIG. 5, and then the fine liquid droplets were also alternately applied to the non-treated grids in the pattern which were skipped in the previous operation, in ascending order of the numbers shown in FIG. 5.

### (Step 2)

Next, the artificial skin model to which the fine liquid droplets were applied was dried in a constant temperature oven set at 40 °C for 10 minutes, and evaluated for a wrinkle improving effect thereof by the following method. The results are shown in Tables 1 to 4.

Incidentally, in the case where the pattern printing was conducted by a plurality of printing operations, the step 2 was conducted after completion of every printing operation.

### Comparative Example 1

The same procedure as in Example 1 was repeated except that the liquid composition was changed to that shown in Table 1 to evaluate the wrinkle improving effect. The results are shown in Table 1.

### Comparative Example 2

Using the liquid composition 1 shown in Table 1 and a microsyringe available from Hamilton Company, the fine liquid droplets of the liquid composition whose mass was 30,000 µg as measured per one droplet thereof were applied to the aforementioned artificial skin model "BIOSKIN, MODEL OF WRINKLES AT OUTER CORNER OF EYE" (step 1'), and then dried by the same method as used in Example 1 (step 2). Next, the wrinkle improving effect was evaluated by the following method. The results are shown in Table 1.

### <Evaluation>

### (Quantitative Evaluation of Wrinkle Improving Effect)

The samples for evaluation obtained in the aforementioned Examples and Comparative Examples were subjected to image evaluation using an image analyzing software "Image J" available from The National Institutes of Health as a quantitative evaluation for the effect of improving wrinkles at an outer corner of the eye.

First, the respective samples for evaluation obtained in the aforementioned Examples and Comparative Examples were placed at the center of a standard light source booth using a standard light source device "Mcbeth Judge II" (light source: D65 lamp) available from Videojet X-Rite K.K., to photograph each sample using a digital camera.

Next, using the "Image J", the thus photographed image was converted into a 8-bit gray-scale image, and then a region (2280 pixels in lateral direction x 2790 pixels in longitudinal direction) in which the wrinkle model was formed was retrieved from the 8-bit gray-scale image to extract a histogram data for contrasting density. The obtained data had 256 gradations including from 0 to 255 gradation levels, and the number of pixels was allocated to each corresponding contrasting density. The contrasting density value of a darker portion became close to 0, whereas the contrasting density value of a lighter portion became close to 255. In addition, since the total number of pixels of the obtained data exceeded 10,000 pixels, the number of pixels was divided by 10,000 to obtain the number per 10,000 pixels, which was then rounded off to the nearest whole number to handle the data as an integer.

In the aforementioned photographing process, in order to enhance reliability of the image evaluation, the intensity of the light source was suitably controlled under the premise that if the pixels indicating 0 or 255 as the numerical value of the histogram for contrasting density were detected, the detection of 0 showed excessively dark condition of the light source, whereas the detection of 255 showed excessively light condition of the light source. As a result, it was confirmed that when the samples for evaluation obtained in the aforementioned Examples and Comparative Examples were photographed, the pixels indicating the numerical value in the range of 0 to 5 or the range of 235 to 255 were not observed.

In addition, in order to confirm the individual difference between the respective artificial skin models used, the number of the pixels was measured every artificial skin model under the same light exposure conditions to determine an average value and a magnitude of fluctuation thereof. As a result, it was confirmed that the magnitude of fluctuation from the average value of the numbers of the pixels fell within the range of ±5%. Moreover, the artificial skin models before being subjected to the skin treatment were also photographed using the digital camera by the same method as used for the samples for evaluation obtained in the aforementioned Examples and Comparative Examples, and the thus photographed images were respectivey converted into a 8-bit gray-scale image, and further the number of pixels allocated thereto was divided by 10,000 and converted into the number per 10,000 pixels.

With respect to the obtained data, the total number of pixels included within the contrasting density range of 0 to 20, the total number of pixels included within the contrasting density range of 0 to 30 and the total number of pixels included within the contrasting density range of 0 to 40 were respectively calculated.

The most important index of the wrinkles is the total number of pixels included within the contrasting density range of 0 to 20 in which as to the appearance of the skin, the wrinkles observed darkly were conspicuous. For this reason, the ratio of the total number of pixels included within the contrasting density range of 0 to 20 after being subjected to the skin treatment to the total number of pixels included within the contrasting density range of 0 to 20 before being subjected to the skin treatment as an index of the degree of reduction of the wrinkles was calculated, and defined as a wrinkle improving rate (1) (%), and used to evaluate the wrinkle improving effect. The smaller the value of the wrinkle improving rate (1) became, the lager the degree of reduction of the wrinkles was, and the more excellent the effect of improving the wrinkles at an outer corner of the eye was.

In addition, by the same method as used above for calculation of the wrinkle improving rate (1) (%), the wrinkle improving rate (2) (%) was calculated from the total numbers of pixels included within the contrasting density range of 0 to 30 before and after being subjected to the skin treatment, as well as the wrinkle improving rate (3) (%) was calculated from the total numbers of pixels included within the contrasting density range of 0 to 40 before and after being subjected to the skin treatment, and respectively used to evaluate the wrinkle improving effect. The smaller the respective values of the wrinkle improving rate (2) and the wrinkle improving rate (3) became, the larger the degree of reduction of the wrinkles was, and the more excellent the effect of improving the wrinkles at an outer corner of the eye was.

### (Qualitative Evaluation of wrinkle Improving Effect)

The samples for evaluation obtained in the aforementioned Examples and Comparative Examples were subjected to qualitative evaluation in which the respective samples were visually observed to evaluate the degree of effect of improving fine wrinkles at an outer corner of the eye on the samples.

### [Evaluation Ratings]

5: Fine wrinkles at an outer corner of the eye were not observed at all, and the appearance of the skin after being treated was natural.
4: Fine wrinkles at an outer corner of the eye became considerably thinned, and the appearance of the skin after being treated was natural.
3: Fine wrinkles at an outer corner of the eye became slightly thinned, and the appearance of the skin after being treated was natural.
2: Fine wrinkles at an outer corner of the eye became almost unthinned, and the appearance of the skin after being treated was kept unchanged from that of the skin before being treated.
1: Fine wrinkles at an outer corner of the eye were not thinned at all or the amount of the fine wrinkles was rather increased than that before being treated, and the appearance of the skin after being treated was unnatural.

### (Quantitative Evaluation of Persistent Wrinkle Improving Effect)

The samples for evaluation obtained in the aforementioned Examples and Comparative Examples were subjected to quantitative evaluation for the persistent effect of improving wrinkles at an outer corner of the eye, in which after allowing the samples for evaluation to undergo deformaion, the samples were subjected to image evaluation by the same method as used in the aforementioned quantitative evaluation for the wrinkle improving effect.

When subjecting the samples for evaluation to deformation, the respective samples were allowed to stand under the environmental conditions of 25 °C and 50% RH for 10 minutes to control the temperature and humidty thereof, and then wound around a surface-smoothened resin cyclinder having a diameter of 80 mm over 1 second such that the treated printed surface of the respective samples faced outside, followed by subjecting the respective samples to flattening operation over 1 second to return them again to a flat shape. The deformation procedure including the winding operation and the flattening operation was repeated 100 times.

Next, the samples for evaluation which have been subjercted to the above deformation procedure were subjected to image evaluation by the same method as used in the aforementioned quantitative evaluation of the wrinkle improving effect.

Similarly to the aforementioned quantitative evaluation of the wrinkle improving effect, the most important index of the wrinkles is the total number of pixels included within the contrasting density range of 0 to 20 in which the wrinkles observed darkly as the appearance of the skin were conspicuous. For this reason, as an index of the persistent wrinkle improving effect, the ratio of the total number of pixels included within the contrasting density range of 0 to 20 after being subjected to the skin treatment to the total number of pixels included within the contrasting density range of 0 to 20 before being subjected to the skin treatment was calculated to obtain a persistent wrinkle improving rate (4) (%), which was used to evaluate the persistent wrinkle improving effect. The smaller the value of the persistent wrinkle improving rate (4) (%) became, the more excellent the persistent effect of improving the wrinkles at an outer corner of the eye was.

In addition, by the same method as used above for determining the persistent wrinkle improving rate (4) (%), the persistent wrinkle improving rate (5) (%) was calculated from the total numbers of pixels included within the contrasting density range of 0 to 30 before and after being subjected to the skin treatment, as well as the persistent wrinkle improving rate (6) (%) was calculated from the total numbers of pixels included within the contrasting density range of 0 to 40 before and after being subjected to the skin treatment, and respectively used to evaluate the persistent wrinkle improving effect. The smaller the values of the persistent wrinkle improving rate (5) and the persistent wrinkle improving rate (6) became, the more excellent the persistent effect of improving the wrinkles at an outer corner of the eye was.

In addition, in the quantitative evaluation of the persistent wrinkle improving effect, it was preferred that the value of the difference between the wrinkle improving rate (1) and the persistent wrinkle improving rate (4) [persistent wrinkle improving rate (4) - wrinkle improving rate (1)] was small. When the difference value is small, it is indicated that once the wrinkles are improved by the skin treatment, the improved good condition of the skin against the wrinkles can be maintained even when an external force or rapid deformation is applied thereto. Therefore, the values of the difference [persistent wrinkle improving rate (4) - wrinkle improving rate (1)] are also shown in Tables 1 to 4. It is also preferred that the value of the difference [persistent wrinkle improving rate (5) - wrinkle improving rate (2)] as well as the value of the difference [persistent wrinkle improving rate (6) - wrinkle improving rate (3)] are small.

The details of the formulation components used in the aforementioned Examples and Comparative Examples as shown in the respective Tables are as follows.

Incidentally, all of amounts (% by mass) of the other components as shown in the respective Tables are on an as-is basis.
*1: "NBN-30-ID" (molar ratio (e/f) in the following formula (4) = 60/40; an isododecane solution of a (norbornene/tris(trimethylsiloxy) silylnorbornene) copolymer of Mn = 360,000; active ingredient content: 30% by mass) available from Shin-Etsu Chemical Co., Ltd., was dried under reduced pressure at 50 °C for 12 hours, and the resulting solid was used. 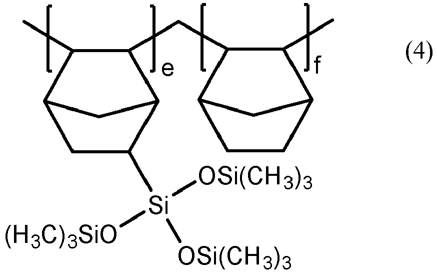
*2: "TSPL-30-ID" (an isododecane solution of tri(trimethylsiloxy)silylpropylcarbamic acid pullulan; active ingredient content: 30% by mass) available from Shin-Etsu Chemical Co., Ltd., was dried under reduced pressure at 50 °C for 12 hours, and the resulting solid was used.
*3: "XS66-B8226" (a cyclopentasiloxane solution of trifluoropropyldimethyl/trimethyl siloxysilicic acid; active ingredient content: 50% by mass) available from Momentive Performance Materials Japan Inc., was dried under reduced pressure at 50 °C for 12 hours, and the resulting solid was used.
*4: "X-21-5595" (an isododecane solution of trimethylsiloxysilicic acid; active ingredient content: 60% by mass) available from Shin-Etsu Chemical Co., Ltd., was dried under reduced pressure at 50 °C for 12 hours, and the resulting solid was used.
*5: Polymethacrylic acid obtained in Preparation Example 1.
*6: Sodium polystyrene sulfonate (weight-average molecular weight: 70,000) available from Alfa Aesar.
*7: "KP-550" (an isododecane solution of a graft copolymer constituted of an acrylic polymer and dimethyl polysiloxane; active ingredient content: 40% by mass) available from Shin-Etsu Chemical Co., Ltd., was dried under reduced pressure at 50 °C for 12 hours, and the resulting solid was used.
*8: "KF-96L-0.65cs" available from Shin-Etsu Chemical Co., Ltd.
*9: "COSMOL 42V" available from Nisshin Oillio Group, Ltd.
*10: "SALACOS 913" available from Nisshin Oillio Group, Ltd.
*11: "SALACOS 334" available from Nisshin Oillio Group, Ltd.
*12: "ELDEW CL-301" available from Ajinomoto, Co., Inc.
*13: "ELDEW PS-306" available from Ajinomoto, Co., Inc.
*14: "PARLEAM EX" available from NOF Corporation.
*15: "NOMCORT W" available from Nisshin Oillio Group, Ltd.
*16: "KF-96A-6cs" available from Shin-Etsu Chemical Co., Ltd.
*17: "KSP-100" available from Shin-Etsu Chemical Co., Ltd.
*18: "KSG-42A" (an isododecane solution; active ingredient content: 20% by mass) available from Shin-Etsu Chemical Co., Ltd.
*19: "KSG-210" (a solution of dimethyl polysiloxane (dynamic viscosity: 6 mm²/s); active ingredient content: 25% by mass) available from Shin-Etsu Chemical Co., Ltd.
*20: "KSG-320" (an isododecane solution; active ingredient content: 25% by mass) available from Shin-Etsu Chemical Co., Ltd.
*21: "TREFIL E-506S" available from Dow Corning Toray Co., Ltd.
*22: "KSG-16" (a solution of dimethyl polysiloxane (dynamic viscosity: 6 mm²/s); active ingredient content: 25% by mass) available from Shin-Etsu Chemical Co., Ltd.

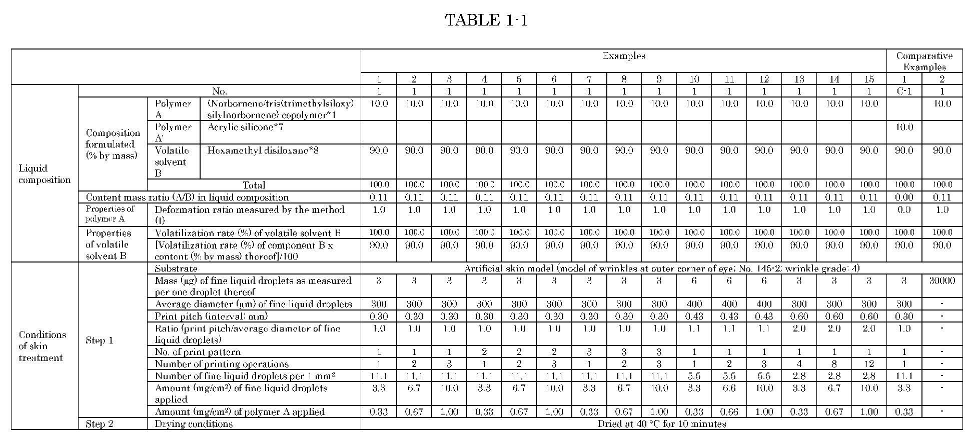

**TABLE 1-2**

| | | | Examples | | | | | | | | | | | | | | | Comparative Examples | |
|---|---|---|---|---|---|---|---|---|---|---|---|---|---|---|---|---|---|---|---|
| | | | 1 | 2 | 3 | 4 | 5 | 6 | 7 | 8 | 9 | 10 | 11 | 12 | 13 | 14 | 15 | 1 | 2 |
| Evaluation | Wrinkle improving rate (1) (%) | Contrasting density in the range of 0 to 20 | 24 | 12 | 0 | 18 | 6 | 0 | 0 | 0 | 0 | 35 | 29 | 18 | 0 | 0 | 0 | 106 | 200 |
| | Wrinkle improving rate (2) (%) | Contrasting density in the range of 0 to 30 | 33 | 25 | 15 | 28 | 21 | 13 | 19 | 13 | 11 | 45 | 36 | 28 | 18 | 12 | 9 | 97 | 99 |
| | Wrinkle improving rate (3) (%) | Contrasting density in the range of 0 to 40 | 36 | 30 | 20 | 33 | 25 | 18 | 30 | 24 | 17 | 51 | 40 | 35 | 23 | 21 | 18 | 103 | 176 |
| | Qualitative evaluation of wrinkle improving effect | | 4 | 4 | 5 | 4 | 4 | 5 | 4 | 5 | 5 | 3 | 3 | 4 | 5 | 5 | 5 | 1 | 1 |
| | Persistent wrinkle improving rate (4) (%) | Contrasting density in the range of 0 to 20 | 29 | 18 | 0 | 24 | 6 | 0 | 6 | 0 | 0 | 47 | 35 | 24 | 0 | 0 | 0 | 106 | 182 |
| | Persistent wrinkle improving rate (5) (%) | Contrasting density in the range of 0 to 30 | 43 | 31 | 18 | 33 | 25 | 15 | 21 | 14 | 11 | 58 | 46 | 35 | 18 | 12 | 9 | 99 | 100 |
| | Persistent wrinkle improving rate (6) (%) | Contrasting density in the range of 0 to 40 | 47 | 38 | 25 | 39 | 30 | 21 | 33 | 26 | 18 | 66 | 51 | 43 | 24 | 21 | 18 | 98 | 162 |
| | Difference [persistent wrinkle improving rate (4) - wrinkle improving rate (1)] | | 5 | 6 | 0 | 6 | 0 | 0 | 6 | 0 | 0 | 12 | 6 | 6 | 0 | 0 | 0 | 0 | -18 |

From Table 1, it was confirmed that in Examples 1 to 15, the fine wrinkles at an outer corner of the eye hardly became conspicuous either quantitatively or qualitatively, and the appearance of the skin after being treated was maintained in a good condition, as compared to Comparative Examples 1 and 2.

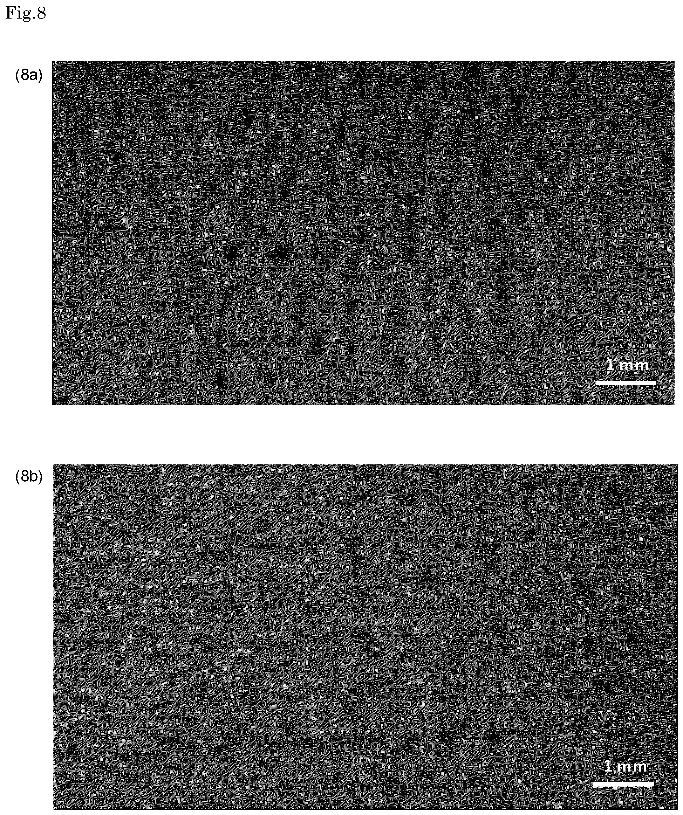

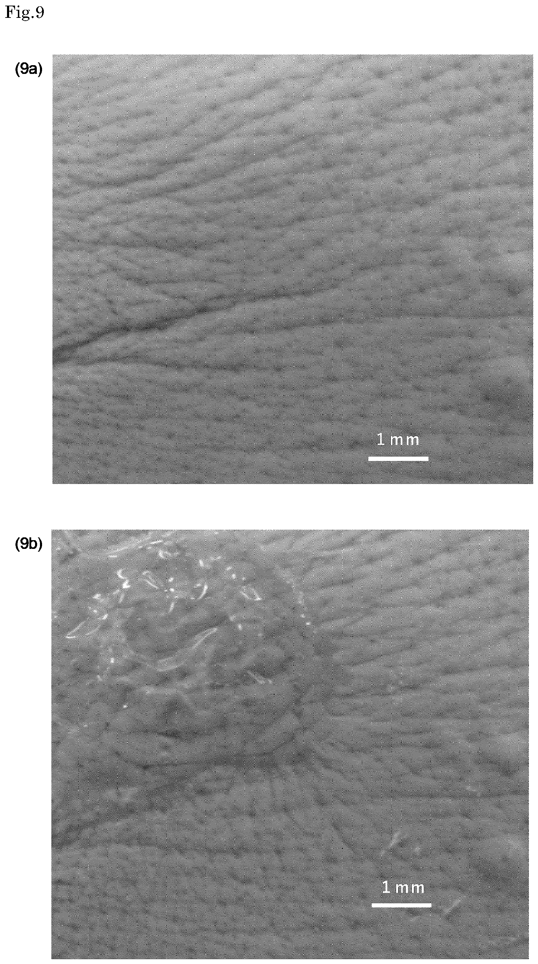

As to the wrinkle-improving effect, the results of Example 13 are shown in FIG. 8, and the results of Comparative Example 2 are shown in FIG. 9. FIG. 8(8a) and FIG. 9(9a) are each a photograph in the peripheral portions of the wrinkles before being subjected to the skin treatment, whereas FIG. 8(8b) and FIG. 9(9b) are each a photograph in the peripheral portions of the wrinkles after being subjected to the skin treatment. As shown in FIG. 8, in Example 13, the fine wrinkles in the peripheral portions of the wrinkles became inconspicuous, whereas as shown in FIG. 9, in Comparative Example 2, it was confirmed that additional wrinkles that were unnatural in appearance and not present before being subjected to the skin treatment were newly formed, and fine concavo-convex unevenness occurred on the surface of the coating film.

**TABLE 2-1**

| | | | | Examples | | | | | |
|---|---|---|---|---|---|---|---|---|---|
| | | | | 16 | 17 | 18 | 19 | 20 | 21 |
| Liquid composition | No. | | | 2 | 3 | 4 | 5 | 6 | 7 |
| | Composition formulated (% by mass) | Polymer A | (Norbornene/tris(trimethylsiloxy)silylnorbomene) copolymer*1 | | | | | | 13.0 |
| | | | Silicone-modified pullulan*2 | 10.0 | | | | | |
| | | | Trifluoropropyldimethyl/trimethylsiloxysilicic acid*3 | | 10.0 | | | | |
| | | | Trimethylsiloxysilicic acid*4 | | | 10.0 | | | |
| | | | Polymethacrylic acid*5 | | | | 10.0 | | |
| | | | Sodium polystyrene sulfonate*6 | | | | | 10.0 | |
| | | Volatile solvent B | Hexamethyl disiloxane*8 | 90.0 | 90.0 | 90.0 | | | 28.8 |
| | | | Isododecane | | | | | | 58.2 |
| | | | Ethanol | | | | 90.0 | | |
| | | | Ion-exchanged water | | | | | 90.0 | |
| | | Total | | 100.0 | 100.0 | 100.0 | 100.0 | 100.0 | 100.0 |
| | Content mass ratio (A/B) in liquid composition | | | 0.11 | 0.11 | 0.11 | 0.11 | 0.11 | 0.15 |
| | Properties of polymer A | Deformation ratio measured by the method (I) | | 0.9 | 0.7 | 0.8 | 1.0 | 1.0 | 1.0 |
| | Properties of volatile solvent B | Volatilization rate (%) of volatile solvent B | | 100.0 | 100.0 | 100.0 | 100.0 | 100.0 | 51.0 |
| | | [Volatilization rate (%) of component B x content (% bv mass) thereof]/100 | | 90.0 | 90.0 | 90.0 | 90.0 | 90.0 | 44.4 |

**TABLE 2-2**

| | | | Examples | | | | | |
|---|---|---|---|---|---|---|---|---|
| | | | 16 | 17 | 18 | 19 | 20 | 21 |
| Conditions of skin treatment | Step 1 | Substrate | Artificial skin model (model of wrinkles at outer corner of eye; No. 145-2; wrinkle grade: 4) | | | | | |
| | | Mass (µg) of fine liquid droplets as measured per one droplet thereof | 3 | 3 | 3 | 3 | 3 | 3 |
| | | Average diameter (µm) of fine liquid droplets | 300 | 300 | 300 | 300 | 300 | 300 |
| | | Print pitch (interval: mm) | 0.60 | 0.60 | 0.60 | 0.60 | 0.60 | 0.60 |
| | | Ratio (print pitch/average diameter of fine liquid droplets) | 2.0 | 2.0 | 2.0 | 2.0 | 2.0 | 2.0 |
| | | No. of print pattern | 1 | 1 | 1 | 1 | 1 | 1 |
| | | Number of printing operations | 4 | 4 | 4 | 4 | 4 | 3 |
| | | Number of fine liquid droplets per 1 mm² | 2.8 | 2.8 | 2.8 | 2.8 | 2.8 | 2.8 |
| | | Amount (mg/cm²) of fine liquid droplets applied | 3.3 | 3.3 | 3.3 | 3.3 | 3.3 | 2.5 |
| | | Amount (mg/cm²) of polymer A applied | 0.33 | 0.33 | 0.33 | 0.33 | 0.33 | 0.33 |
| | Step 2 | Drying conditions | Dried at 40 °C for 10 minutes | | | | | |
| Evaluation | Wrinkle improving rate (1) (%) | Contrasting density in the range of 0 to 20 | 18 | 29 | 35 | 0 | 0 | 18 |
| | Wrinkle improving rate (2) (%) | Contrasting density in the range of 0 to 30 | 21 | 37 | 44 | 9 | 10 | 31 |
| | Wrinkle improving rate (3) (%) | Contrasting density in the range of 0 to 40 | 29 | 40 | 50 | 16 | 17 | 35 |
| | Qualitative evaluation of wrinkle improving effect | | 4 | 3 | 3 | 5 | 5 | 4 |
| | Persistent wrinkle improving rate (4) (%) | Contrasting density in the range of 0 to 20 | 24 | 35 | 47 | 47 | 53 | 18 |
| | Persistent wrinkle improving rate (5) (%) | Contrasting density in the range of 0 to 30 | 25 | 48 | 57 | 58 | 60 | 32 |
| | Persistent wrinkle improving rate (6) (%) | Contrasting density in the range of 0 to 40 | 35 | 53 | 65 | 66 | 69 | 36 |
| | Difference [persistent wrinkle improving rate (4) - wrinkle improving rate (1)] | | 6 | 6 | 12 | 47 | 53 | 0 |

From Table 2, it was confirmed that in Examples 16 to 21, the fine wrinkles at an outer corner of the eye hardly became conspicuous either quantitatively or qualitatively irrespective of the kind of polymer A or the change in kind of volatile solvent B.

**TABLE 3-1**

| | | | | Examples | | | | | | | | |
|---|---|---|---|---|---|---|---|---|---|---|---|---|
| | | | | 22 | 23 | 24 | 25 | 26 | 27 | 28 | 29 | 30 |
| | No. | | | 8 | 9 | 10 | 11 | 12 | 13 | 14 | 15 | 16 |
| | | Polymer A | (Norbornene/tris(trimethylsiloxy) silylnorbornene) copolymer*1 | 13.0 | 13.0 | 13.0 | 13.0 | 13.0 | 13.0 | 13.0 | 13.0 | |
| | | | Silicone-modified pullulan*2 | | | | | | | | | 13.0 |
| | | | Trifluoropropyldimethyl/trimethylsiloxysilicic acid*3 | | | | | | | | | |
| | | | Trimethylsiloxysilicic acid*4 | | | | | | | | | |
| | | Volatile solvent B | Hexamethyl disiloxane*8 | 55.7 | 55.7 | 55.7 | 55.7 | 55.7 | 55.7 | 55.7 | 55.7 | 55.7 |
| | | | Isododecane | 30.3 | 30.3 | 30.3 | 30.3 | 30.3 | 30.3 | 30.3 | 30.3 | 30.3 |
| | | | Ethanol | | | | | | | | | |
| | | | Ion-exchanged water | | | | | | | | | |
| | | | Polyglyceryl-2 diisostearate*9 | 1.0 | | | | | | | | 1.0 |
| | | | Isotridecyl isononanoate*10 | | 1.0 | | | | | | | |
| | | | Glyceryl tri(caprylate/caprinate/myristate/stearate)*11 | | | 1.0 | | | | | | |
| Liquid composition | Composition formulated (% by mass) | | Di(cholesteryl/behenyl/octyldodecyl) lauroyl glutamate*12 | | | | 1.0 | | | | | |
| | | | Di(octyldodecyl/phytosteryl/behenyl) lauroyl glutamate*13 | | | | | 1.0 | | | | |
| | | | Hydrogenated polvisobutene*14 | | | | | | 1.0 | | | |
| | | | Vaseline*15 | | | | | | | 1.0 | | |
| | | Other components | Dimethyl polysiloxane (dynamic viscosity: 6 mm²/s)*16 | | | | | | | | 1.0 | |
| | | | (Vinyl dimethicone/methicone silsesquioxane) crosspolymer*17 | | | | | | | | | |
| | | | (Vinyl dimethicone/lauryl dimethicone) crosspolymer*18 | | | | | | | | | |
| | | | (Dimethicone/(PEG-10/15)) crosspolymer*19 | | | | | | | | | |
| | | | (PEG-15/lauryl dimethicone) crosspolymer*20 | | | | | | | | | |
| | | | (Dimethicone/vinyl dimethicone) crosspolymer*21 | | | | | | | | | |
| | | | (Dimethicone/vinyl dimethicone) crosspolymer*22 | | | | | | | | | |
| | | Total | | 100.0 | 100.0 | 100.0 | 100.0 | 100.0 | 100.0 | 100.0 | 100.0 | 100.0 |

**TABLE 3-2**

| | | | | Examples | | | | | | | | |
|---|---|---|---|---|---|---|---|---|---|---|---|---|
| | | | | 22 | 23 | 24 | 25 | 26 | 27 | 28 | 29 | 30 |
| Liquid composition | Content mass ratio (A/B) in liquid composition | | | 0.15 | 0.15 | 0.15 | 0.15 | 0.15 | 0.15 | 0.15 | 0.15 | 0.15 |
| | Content mass ratio (A/other components) in liquid composition | | | 13.0 | 13.0 | 13.0 | 13.0 | 13.0 | 13.0 | 13.0 | 13.0 | 13.0 |
| | Properties of polymer A | Deformation ratio measured by the method (I) | | 1.0 | 1.0 | 1.0 | 1.0 | 1.0 | 1.0 | 1.0 | 1.0 | 0.9 |
| | Properties of volatile solvent B | Volatilization rate (%) of volatile solvent B | | 79.4 | 79.4 | 79.4 | 79.4 | 79.4 | 79.4 | 79.4 | 79.4 | 79.4 |
| | | [Volatilization rate (%) of component B x content (% by mass) thereofl/100 | | 68.3 | 68.3 | 68.3 | 68.3 | 68.3 | 68.3 | 68.3 | 68.3 | 68.3 |
| Conditions of skin treatment | Step 1 | Substrate | | Artificial skin model (model of wrinkles at outer corner of eye; No. 145-2; wrinkle grade: 4) | | | | | | | | |
| | | Mass (µg) of fine liquid droplets as measured per one droplet thereof | | 3 | 3 | 3 | 3 | 3 | 3 | 3 | 3 | 3 |
| | | Average diameter (µm) of fine liquid droplets | | 300 | 300 | 300 | 300 | 300 | 300 | 300 | 300 | 300 |
| | | Print pitch (interval: mm) | | 0.60 | 0.60 | 0.60 | 0.60 | 0.60 | 0.60 | 0.60 | 0.60 | 0.60 |
| | | Ratio (print pitch/average diameter of fine liquid droplets) | | 2.0 | 2.0 | 2.0 | 2.0 | 2.0 | 2.0 | 2.0 | 2.0 | 2.0 |
| | | No. of print pattern | | 1 | 1 | 1 | 1 | 1 | 1 | 1 | 1 | 1 |
| | | Number of printing operations | | 3 | 3 | 3 | 3 | 3 | 3 | 3 | 3 | 3 |
| | | Number of fine liquid droplets per 1 mm² | | 2.8 | 2.8 | 2.8 | 2.8 | 2.8 | 2.8 | 2.8 | 2.8 | 2.8 |
| | | Amount (mg/cm²) of fine liquid droplets applied | | 2.5 | 2.5 | 2.5 | 2.5 | 2.5 | 2.5 | 2.5 | 2.5 | 2.5 |
| | | Amount (mg/cm²) of polymer A applied | | 0.33 | 0.33 | 0.33 | 0.33 | 0.33 | 0.33 | 0.33 | 0.33 | 0.33 |
| | Step 2 | Drying conditions | | Dried at 40 °C for 10 minutes | | | | | | | | |
| Evaluation | Wrinkle impr (%) oving rate (1) | | Contrasting density in the range of 0 to 20 | 0 | 0 | 0 | 0 | 0 | 0 | 0 | 0 | 24 |
| | Wrinkle impr (%) oving rate (2) | | Contrasting density in the range of 0 to 30 | 11 | 15 | 12 | 11 | 17 | 14 | 12 | 17 | 33 |
| | Wrinkle impr (%) oving rate (3) | | Contrasting density in the range of 0 to 40 | 24 | 21 | 20 | 23 | 21 | 20 | 23 | 20 | 35 |
| | Qualitative evaluation of wrinkle improving effect | | | 5 | 5 | 5 | 5 | 5 | 5 | 5 | 5 | 4 |
| | Persistent wr rate (4) (%) inkle improving | | Contrasting density in the range of 0 to 20 | 0 | 0 | 0 | 0 | 0 | 0 | 0 | 0 | 29 |
| | Persistent wr rate (5) (%) inkle improving | | Contrasting density in the range of 0 to 30 | 11 | 15 | 13 | 12 | 17 | 15 | 12 | 17 | 38 |
| | Persistent wr rate (6) (%) inkle improving | | Contrasting density in the range of 0 to 40 | 25 | 22 | 22 | 25 | 22 | 21 | 24 | 21 | 40 |
| | Difference [persistent wrinkle improving rate (4) - wrinkle improving rate (1)] | | | 0 | 0 | 0 | 0 | 0 | 0 | 0 | 0 | 5 |

**TABLE 4-1**

| | | | | Examples | | | | | | | | |
|---|---|---|---|---|---|---|---|---|---|---|---|---|
| | | | | 31 | 32 | 33 | 34 | 35 | 36 | 37 | 38 | 39 |
| | No. | | | 17 | 18 | 19 | 20 | 21 | 22 | 23 | 24 | 25 |
| | | Polymer A | (Norbornene/tris(trimethylsiloxy) silylnorbornene) copolymer*1 | | 13.0 | 13.0 | 13.0 | 13.0 | 13.0 | 13.0 | | |
| | | | Silicone-modified pullulan*2 | | | | | | | | 13.0 | |
| | | | Trifluoropropyldimethyl/trimethylsiloxysilicic acid*3 | | | | | | | | | |
| | | | Trimethylsiloxysilicic acid*4 | 13.0 | | | | | | | | 13.0 |
| | | Volatile solvent B | Hexamethyl disiloxane*8 | 55.7 | 52.7 | 52.7 | 52.7 | 52.7 | 52.7 | 52.7 | 52.7 | 52.7 |
| | | | Isododecane | 30.3 | 30.3 | 30.3 | 30.3 | 30.3 | 32.9 | 30.3 | 30.3 | 30.3 |
| | | | Ethanol | | | | | | | | | |
| | | | Ion-exchanged water | | | | | | | | | |
| | | | Polyglyceryl-2 diisostearate*9 | 1.0 | | | | | | | | |
| | | | Isotridecyl isononanoate*10 | | | | | | | | | |
| | | | Glyceryl tri(caprylate/caprinate/myristate/stearate)*11 | | | | | | | | | |
| Liquid composition | Composition formulated (% by mass) | | Di(cholesteryl/behenyl/octyldodecyl) lauroyl glutamate*12 | | | | | | | | | |
| | | | Di(octyldodecyl/phytosteryl/behenyl) lauroyl glutamate*13 | | | | | | | | | |
| | | | Hydrogenated polyisobutene*14 | | | | | | | | | |
| | | | Vaseline*15 | | | | | | | | | |
| | | Other components | Dimethyl polysiloxane (dynamic viscosity: 6 mm²/s)*16 | | | | | | | | | |
| | | | (Vinyl dimethicone/methicone silsesquioxane) crosspolymer*17 | | 4.0 | | | | | | 4.0 | 4.0 |
| | | | (Vinyl dimethicone/lauryl dimethicone) crosspolymer*18 | | | 4.0 | | | | | | |
| | | | (Dimethicone/(PEG-10/15)) crosspolymer*19 | | | | 4.0 | | | | | |
| | | | (PEG-15/lauryl dimethicone) crosspolymer*20 | | | | | 4.0 | | | | |
| | | | (Dimethicone/vinyl dimethicone) crosspolymer*21 | | | | | | 1.4 | | | |
| | | | (Dimethicone/vinyl dimethicone) crosspolymer*22 | | | | | | | 4.0 | | |
| | | Total | | 100.0 | 100.0 | 100.0 | 100.0 | 100.0 | 100.0 | 100.0 | 100.0 | 100.0 |

**TABLE 4-2**

| | | | | Examples | | | | | | | | |
|---|---|---|---|---|---|---|---|---|---|---|---|---|
| | | | | 31 | 32 | 33 | 34 | 35 | 36 | 37 | 38 | 39 |
| Liquid composition | Content (% by mass) of volatile solvent B in liquid composition | | | 86.0 | 83.0 | 86.2 | 83.0 | 86.0 | 85.6 | 83.0 | 83.0 | 83.0 |
| | Content mass ratio (A/B) in liquid composition | | | 0.15 | 0.16 | 0.15 | 0.16 | 0.15 | 0.15 | 0.16 | 0.16 | 0.16 |
| | Content mass ratio (A/other components) in liquid composition | | | 13.0 | 3.3 | 16.3 | 3.3 | 13.0 | 9.3 | 3.3 | 3.3 | 3.3 |
| | Properties of polymer A | Deformation ratio measured by the method (1) | | 0.8 | 1.0 | 1.0 | 1.0 | 1.0 | 1.0 | 1.0 | 0.9 | 0.8 |
| | Properties of volatile solvent B | Volatilization rate (%) of volatile solvent B | | 79.4 | 78.6 | 78.6 | 78.6 | 78.6 | 78.6 | 78.6 | 78.6 | 78.6 |
| | | [Volatilization rate (%) of component B x content (% by mass) thereof]/100 | | 68.3 | 65.2 | 652 | 652 | 65.2 | 67.3 | 652 | 65.2 | 652 |
| Conditions of skin treatment | Step 1 | Substrate | | Artificial skin model (model of wrinkles at outer corner of eye; No. 145-2; wrinkle grade: 4) | | | | | | | | |
| | | Mass (µg) of fine liquid droplets as measured per one droplet thereof | | 3 | 3 | 3 | 3 | 3 | 3 | 3 | 3 | 3 |
| | | Average diameter (µm) of fine liquid droplets | | 300 | 300 | 300 | 300 | 300 | 300 | 300 | 300 | 300 |
| | | Print pitch (interval: mm) | | 0.60 | 0.60 | 0.60 | 0.60 | 0.60 | 0.60 | 0.60 | 0.60 | 0.60 |
| | | Ratio (print pitch/average diameter of fine liquid droplets) | | 2.0 | 2.0 | 2.0 | 2.0 | 2.0 | 2.0 | 2.0 | 2.0 | 2.0 |
| | | No. of print pattern | | 1 | 1 | 1 | 1 | 1 | 1 | 1 | 1 | 1 |
| | | Number of printing operations | | 3 | 3 | 3 | 3 | 3 | 3 | 3 | 3 | 3 |
| | | Number of fine liquid droplets per 1 mm² | | 2.8 | 2.8 | 2.8 | 2.8 | 2.8 | 2.8 | 2.8 | 2.8 | 2.8 |
| | | Amount (mg/cm²) of fine liquid droplets applied | | 2.5 | 2.5 | 2.5 | 2.5 | 2.5 | 2.5 | 2.5 | 2.5 | 2.5 |
| | | Amount (mg/cm²) of polymer A applied | | 0.33 | 0.33 | 0.33 | 0.33 | 0.33 | 0.33 | 0.33 | 0.33 | 0.33 |
| | Step 2 | Drying conditions | | Dried at 40 °C for 10 minutes | | | | | | | | |
| Evaluation | Wrinkle improving rate (1) (%) | | Contrasting density in the range of 0 to 20 | 29 | 0 | 0 | 0 | 0 | 0 | 0 | 18 | 29 |
| | Wrinkle improving rate (2) (%) | | Contrasting density in the range of 0 to 30 | 37 | 14 | 13 | 16 | 13 | 16 | 15 | 27 | 36 |
| | Wrinkle improving rate (3) (%) | | Contrasting density in the range of 0 to 40 | 40 | 22 | 22 | 20 | 21 | 22 | 24 | 28 | 40 |
| | Qualitative evaluation of wrinkle improving effect | | | 3 | 5 | 5 | 5 | 5 | 5 | 5 | 4 | 3 |
| | Persistent wrinkle improving rate (4) (%) | | Contrasting density in the range of 0 to 20 | 41 | 0 | 0 | 0 | 0 | 0 | 0 | 24 | 41 |
| | Persistent wrinkle improving rate (5) (%) | | Contrasting density in the range of 0 to 30 | 48 | 14 | 13 | 17 | 14 | 16 | 16 | 32 | 47 |
| | Persistent wrinkle improving rate (6) (%) | | Contrasting density in the range of 0 to 40 | 53 | 24 | 23 | 21 | 22 | 23 | 25 | 33 | 52 |
| | Difference [persistent wrinkle improving rate (4) - wrinkle improving rate (1)] | | | 12 | 0 | 0 | 0 | 0 | 0 | 0 | 6 | 12 |

From Tables 3 and 4, it was confirmed that in Examples 22 to 39, the fine wrinkles at an outer corner of the eye hardly became conspicuous either quantitatively or qualitatively irrespective of addition or non-addition of the optional components.

### Examples 40 to 54 and Comparative Example 3

### (Step 1)

Using the respective liquid compositions and conditions shown in Table 5, pattern printing was performed on an area having a longitudinal length of 5.6 mm and a lateral length of 10.4 mm on an artificial skin model "BIOSKIN, MODEL OF WRINKLES AT OUTER CORNER OF EYE" (product number : No. 145-2; wrinkle grade: 8) as a replica sample of skin available from Beaulax Co., Ltd., to apply fine liquid droplets of the respective liquid compositions thereto. Incidentally, the pattern printing was conducted along opposite ends of the deep wrinkles present at an outer corner of the eye.

In Examples 40 to 51, as a pattern printing apparatus, there was used a handy ink-jet printer "KGKJET HQ1000H" available from KISHU GIKEN KOGYO CO., LTD., which was modified with "PRO4" available from Nordson Corporation so as to control a print pitch upon the printing. An interior of an ink cartridge "TK403 BLACK-CS CARTRIDGE" available from KISHU GIKEN KOGYO CO., LTD., used in the printer was washed with ion-exchanged water and ethanol, and the ink cartridge was filled with ion-exchanged water having an electric conductivity of not more than 0.6 µS/cm. Then, the ion-exchanged water thus filled in the ink cartridge was ejected from an ink-jet head of the printer and collected, and after confirming that the electric conductivity of the ion-exchanged water collected became not more than 1.0 µS/cm, the interior of the ink cartridge was evacuated and dried by a vacuum dryer before being used in the printer.

In Examples 52 to 54 and Comparative Example 3, as a pattern printing apparatus, there was used the same piezoelectric jet dispenser as used in Example 1.

Meanwhile, the mass of the fine liquid droplets as measured per one droplet thereof as well as the average diameter of the fine liquid droplets applied and the print pitch as shown in Table 5 were determined by the same methods as described above. The respective numbers of the print patterns shown in Table 5 were also the same as those described above. (Step 2)

Next, the artificial skin model to which the fine liquid droplets were applied was dried in a constant temperature oven set at 40 °C for 10 minutes, and evaluated for a wrinkle improving effect and a persistent wrinkle improving effect thereof by the same methods as described above. The results are shown in Table 5.

Incidentally, in the case where the pattern printing was conducted by a plurality of printing operations, the step 2 was conducted after completion of every printing operation.

**TABLE 5-1**

| | | | | Examples | | | | | | | | | | | | | | | Com. Ex. |
|---|---|---|---|---|---|---|---|---|---|---|---|---|---|---|---|---|---|---|---|
| | | | | 40 | 41 | 42 | 43 | 44 | 45 | 46 | 47 | 48 | 49 | 50 | 51 | 52 | 53 | 54 | 3 |
| Liquid composition | No. | | | 5 | 5 | 5 | 5 | 5 | 5 | 5 | 5 | 5 | 5 | 5 | 5 | 1 | 1 | 1 | C-1 |
| | Composition formulated (% by mass) | Polymer A | (N orhornene/tris (trimethylsiloxy) silvlnorbornene) copolymer*1 | | | | | | | | | | | | | 10.0 | 10.0 | 10.0 | |
| | | | Polymethacrylic acid*5 | 10.0 | 10.0 | 10.0 | 10.0 | 10.0 | 10.0 | 10.0 | 10.0 | 10.0 | 10.0 | 10.0 | 10.0 | | | | |
| | | Polymer A' | Acrylic silicone*7 | | | | | | | | | | | | | | | | 10.0 |
| | | Volatile solvent B | Hexamethyl disiloxane*8 | | | | | | | | | | | | | 90.0 | 90.0 | 90.0 | 90.0 |
| | | | Ethanol | 90.0 | 90.0 | 90.0 | 90.0 | 90.0 | 90.0 | 90.0 | 90.0 | 90.0 | 90.0 | 90.0 | 90.0 | | | | |
| | | Total | | 100.0 | 100.0 | 100.0 | 100.0 | 100.0 | 100.0 | 100.0 | 100.0 | 100.0 | 100.0 | 100.0 | 100.0 | 100.0 | 100.0 | 100.0 | 100.0 |
| | Content mass ratio (A/B) in liquid composition | | | 0.11 | 0.11 | 0.11 | 0.11 | 0.11 | 0.11 | 0.11 | 0.11 | 0.11 | 0.11 | 0.11 | 0.11 | 0.11 | 0.11 | 0.11 | 0.11 |
| | Properties of polymer A | Deformation ratio measured by the method (I) | | 1.0 | 1.0 | 1.0 | 1.0 | 1.0 | 1.0 | 1.0 | 1.0 | 1.0 | 1.0 | 1.0 | 1.0 | 1.0 | 1.0 | 1.0 | 0.0 |
| | Properties of volatile solvent B | Volatilization rate (%) of volatile solvent B | | 100.0 | 100.0 | 100.0 | 100.0 | 100.0 | 100.0 | 100.0 | 100.0 | 100.0 | 100.0 | 100.0 | 100.0 | 100.0 | 100.0 | 100.0 | 100.0 |
| | | [Volatilization rate (%) of component B x content (% by mass) thereof]/100 | | 90.0 | 90.0 | 90.0 | 90.0 | 90.0 | 90.0 | 90.0 | 90.0 | 90.0 | 90.0 | 90.0 | 90.0 | 90.0 | 90.0 | 90.0 | 90.0 |
| Conditions of skin treatment | Step 1 | Substrate | | Artificial skin model (model of wrinkles at outer corner of eye; No. 145-2; wrrinkle grade: 8) | | | | | | | | | | | | | | | |
| | | Mass (µg) of fine liquid droplets as measured per one droplet thereof | | 0.033 | 0.033 | 0.033 | 0.033 | 0.033 | 0.033 | 0.033 | 0.033 | 0.033 | 0.033 | 0.033 | 0.033 | 4 | 4 | 4 | 4 |
| | | Average diameter (µm) of fine liquid droplets | | 50 | 50 | 50 | 50 | 50 | 50 | 50 | 50 | 50 | 50 | 50 | 50 | 315 | 315 | 315 | 315 |
| | | Print pitch (interval: mm) | | 0.05 | 0.05 | 0.05 | 0.05 | 0.05 | 0.05 | 0.05 | 0.05 | 0.05 | 0.10 | 0.10 | 0.10 | 0.32 | 0.32 | 0.32 | 0.32 |
| | | Ratio (print pitch/average diameter of fine liquid droplets) | | 1.0 | 1.0 | 1.0 | 1.0 | 1.0 | 1.0 | 1.0 | 1.0 | 1.0 | 2.0 | 2.0 | 2.0 | 1.0 | 1.0 | 1.0 | 1.0 |
| | | No. of print pattern | | 1 | 1 | 1 | 2 | 2 | 2 | 3 | 3 | 3 | 1 | 1 | 1 | 1 | 1 | 1 | 1 |
| | | Number of printing operations | | 3 | 6 | 9 | 3 | 6 | 9 | 3 | 6 | 9 | 12 | 24 | 36 | 1 | 2 | 3 | 1 |
| | | Number of fine liquid droplets per 1 mm² | | 400 | 400 | 400 | 400 | 400 | 400 | 400 | 400 | 400 | 100 | 100 | 100 | 10 | 10 | 10 | 10 |
| | | Amount (mg/cm²) of fine liquid droplets applied | | 4.0 | 8.0 | 12.0 | 4.0 | 8.0 | 12.0 | 4.0 | 8.0 | 12.0 | 4.0 | 8.0 | 12.0 | 4.0 | 8.0 | 12.0 | 4.0 |
| | | Amount (mg/cm²) of polymer A applied | | 0.40 | 0.80 | 1.20 | 0.40 | 0.80 | 1.20 | 0.40 | 0.80 | 1.20 | 0.40 | 0.80 | 1.20 | 0.40 | 0.80 | 1.20 | 0.40 |
| | Step 2 | Drying conditions | | Dried at 40 °C for 10 minutes | | | | | | | | | | | | | | | |

**TABLE 5-2**

| | | | Examples | | | | | | | | | | | | | | | Com. Ex. |
|---|---|---|---|---|---|---|---|---|---|---|---|---|---|---|---|---|---|---|
| | | | 40 | 41 | 42 | 43 | 44 | 45 | 46 | 47 | 48 | 49 | 50 | 51 | 52 | 53 | 54 | 3 |
| Evaluation | Wrinkle improving rate (1) (%) | Contrasting density in the range of 0 to 20 | 13 | 7 | 0 | 10 | 3 | 0 | 7 | 0 | 0 | 0 | 0 | 0 | 23 | 13 | 7 | 100 |
| | Wrinkle improving rate (2) (%) | Contrasting density in the range of 0 to 30 | 22 | 16 | 6 | 19 | 15 | 9 | 14 | 9 | 7 | 8 | 7 | 6 | 36 | 22 | 16 | 100 |
| | Wrinkle improving rate (3) (%) | Contrasting density in the range of 0 to 40 | 36 | 33 | 24 | 35 | 33 | 28 | 31 | 28 | 26 | 29 | 26 | 25 | 47 | 37 | 34 | 100 |
| | Qualitative evaluation of wrinkle improving effect | | 4 | 4 | 5 | 4 | 4 | 5 | 4 | 5 | 5 | 5 | 5 | 5 | 3 | 4 | 4 | 1 |
| | Persistent wrinkle improving rate (4) (%) | Contrasting density in the range of 0 to 20 | 17 | 9 | 0 | 12 | 4 | 1 | 9 | 1 | 0 | 1 | 1 | 0 | 25 | 15 | 5 | 102 |
| | Persistent wrinkle improving rate (5) (%) | Contrasting density in the range of 0 to 30 | 28 | 21 | 7 | 22 | 18 | 10 | 15 | 10 | 7 | 10 | 9 | 7 | 38 | 24 | 17 | 97 |
| | Persistent wrinkle improving rate (6) (%) | Contrasting density in the range of 0 to 40 | 47 | 42 | 30 | 42 | 39 | 33 | 34 | 30 | 26 | 38 | 33 | 31 | 49 | 38 | 35 | 99 |
| | Difference [persistent wrinkle improving rate (4) - wrinkle improving rate (1)] | | 4 | 2 | 0 | 2 | 1 | 1 | 2 | 1 | 0 | 1 | 1 | 0 | 2 | 2 | -2 | 2 |

From Table 5, it was confirmed that in Examples 40 to 54, the fine wrinkles at an outer corner of the eye hardly became conspicuous either quantitatively or qualitatively as compared to Comparative Example 3.

As to the wrinkle-improving effect, the results of Example 42 are shown in FIG. 10. FIG. 10(10a) is a photograph in the peripheral portions of the wrinkles on the sample before being subjected to the skin treatment, whereas FIG. 10(10b) is a photograph in the peripheral portions of the wrinkles on the sample after being subjected to the skin treatment. As shown in FIG. 10, it was confirmed that in Example 42, the fine wrinkles within the portion surrounded by a white line frame became inconspicuous.

### <Formation of Eye Bags>

### Examples 55 to 69 and Comparative Example 4

### (Step 1)

Using the respective liquid compositions and conditions shown in Table 6, pattern printing was performed on an area having a longitudinal length of 30 mm and a lateral length of 20 mm on a skin texture reproduced surface of a grain-like artificial leather "SUPULARE PBZ13001" (tradename; available from IDEATEX Japan Co., Ltd.) cut into a size of a width of 3 cm and a length of 4 cm as a replica sample of skin, to apply fine liquid droplets of the respective liquid compositions thereto.

In Examples 55 to 66, as a pattern printing apparatus, there were used the same handy ink-jet printer and ink cartridge as those used in Example 40, and the ink cartridge was washed and dried in the same manner as in Example 40 before being used in the printer.

In Examples 67 to 69 and Comparative Example 4, as a pattern printing apparatus, there was used the same piezoelectric jet dispenser as used in Example 1.

Meanwhile, the mass of the fine liquid droplets as measured per one droplet thereof as well as the average diameter of the fine liquid droplets and the print pitch as shown in Table 6 were determined by the same methods as described above. The print patterns shown in Table 6 are as follows.

Print pattern 4: As shown in FIG. 11, using a rectangular grid pattern, the fine liquid droplets were applied to the respective grids in the pattern from ends thereof along the vertical direction in ascending order of the numbers shown in FIG. 11 to conduct the printing uniformly over a whole surface area of the region to be printed.

Print pattern 5: As shown in FIG. 12, using a rectangular grid pattern, the fine liquid droplets were applied to the respective grids in the pattern from an outer end thereof toward the respective inner grid regions in a spiral manner in ascending order of the numbers shown in FIG. 12 to conduct the printing thereon.

Print pattern 6: As shown in FIG. 13, using a rectangular grid pattern, the fine liquid droplets were alternately applied to the respective grids in the pattern from an outer end thereof toward the respective inner grid regions in a spiral manner in ascending order of the numbers shown in FIG. 13, and then the fine liquid droplets were also alternately applied to the grids in the pattern which were untreated and skipped in the previous operation, in ascending order of the numbers shown in FIG. 13, to conduct the printing thereon.

### (Step 2)

Next, the artificial skin model to which the fine liquid droplets were applied was dried in a constant temperature oven set at 40 °C for 10 minutes, and the resulting sample for evaluation was evaluated for the effect of forming eye bags by the following method. The results are shown in Table 6.

Incidentally, in the case where the pattern printing was conducted by a plurality of printing operations, the step 2 was conducted after completion of every printing operation.

### Comparative Example 5

Using the liquid composition shown in Table 6, the fine liquid droplets of the liquid composition were applied to a skin texture reproduced surface of a grain-like artificial leather "SUPULARE PBZ13001" (tradename; available from IDEATEX Japan Co., Ltd.) cut into a size of a width of 3 cm and a length of 4 cm as a replica sample of skin using the aforementioned microsyringe such that the mass of the fine liquid droplets applied was 30,000 µg as measured per one droplet thereof (step 1'), and then the artificial leather to which the fine liquid droplets were applied was dried in a constant temperature oven set at 40 °C for 10 minutes (step 2).

The wrinkle model on the artificial leather underwent deformation in association with drying of the fine liquid droplets of the liquid composition applied thereto. However, fine concavo-convex unevenness was caused on the surface of the coating film of the liquid composition, so that it was not possible to obtain an intended undulation shape which looked like a raised portion of the artificial leather.

The thus obtained sample for evaluation was evaluated for the effect of forming eye bags by the following method. The results are shown in Table 6.

### <Evaluation>

### (Visual Evaluation of Effect of Forming Eye Bags)

The respective samples for evaluation obtained in the aforementioned Examples and Comparative Examples were visually observed by 5 evaluation panelists to evaluate the degree of the effect of forming eye bags on a scale of five points as a perfect score for each panelist according to the following evaluation ratings. The total values of the obtained scores for the respective samples are shown in Table 6. The larger the total value of the scores became, the more excellent the effect of forming eye bags was.

### [Evaluation Ratings]

5: A very large undulation shape raised like eye bags was obtained, and the appearance of the skin treated looked natural.
4: An undulation shape raised almost equivalently to eye bags was obtained, and the appearance of the skin treated looked natural.
3: A slightly large undulation shape raised like eye bags was confirmed, and the appearance of the skin treated posed substantially no problems.
2: Although slightly raised deformation was recognized on the skin treated, the deformation was insufficient to obtain an undulation shape raised like eye bags, and the appearance of the skin treated looked unnatural, and posed the problems upon practical use.
1: Substantially no raised deformation was observed on the skin treated.

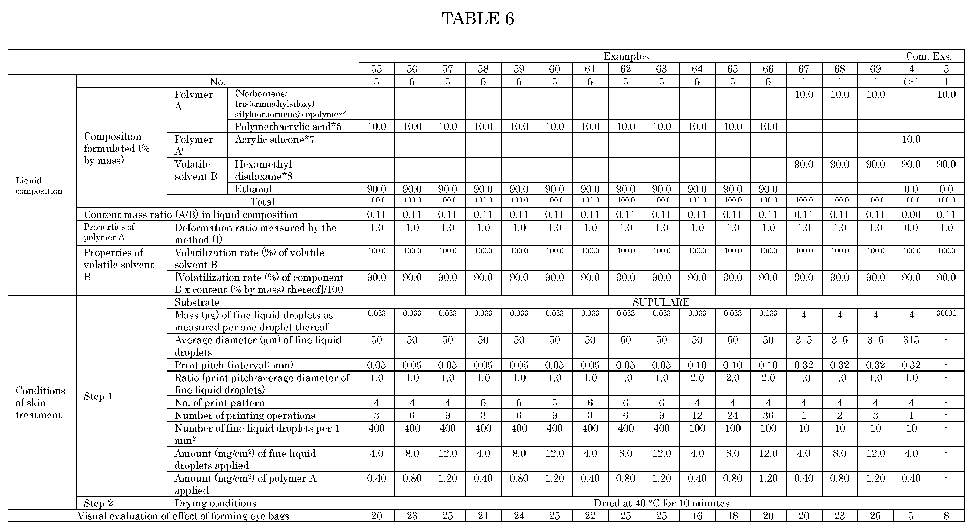

From Table 6, it was confirmed that in Examples 55 to 69, an undulation shape raised like eye bags could be suitably formed on the respective samples treated, as compared to Comparative Examples 4 and 5.

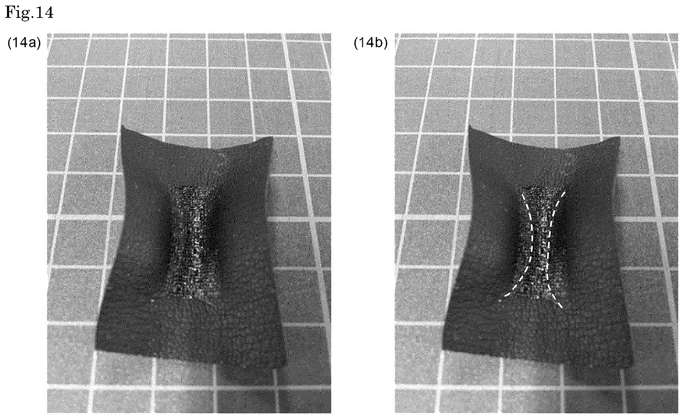

As to the effect of forming eye bags, the results of Example 57 are shown in FIG. 14. FIG. 14(14b) is a photograph made by adding an auxiliary line to a photograph shown in FIG. 14(14a) to indicate a convex portion formed on the sample. As shown in FIG. 14(14b), it was confirmed that in Example 57, the portion of the sample subjected to the skin treatment as viewed along the auxiliary line was formed into a raised undulation shape.

### Industrial Applicability

According to the skin treatment method of the present invention, it is possible to finely control an external shape of skin in an non-invasive manner without damage to natural appearance of the skin. Therefore, the skin treatment method of the present invention can be suitably used to improve wrinkles on the skin, form an undulation shape on the skin, and improve sagging of the skin.

### Reference Signs List

1: Polyethylene sheet
1a: An end of a short side of the polyethylene sheet 1
1': Polyethylene sheet with a polymer film
2: Portion to which a polymer solution is applied
21: Fine liquid droplets
22: Skin

## Claims

1. A skin treatment method comprising:
Step 1: applying fine liquid droplets of a liquid composition comprising a polymer A and a volatile solvent B to skin; and
Step 2: drying the fine liquid droplets applied to the skin in the step 1 to contract the skin in association with the drying, in which:
a deformation ratio of the polymer A as measured by the following method (I) is not less than 0.3 and not more than 1; and
a mass of the fine liquid droplets applied to the skin in the step 1 as measured per one droplet thereof is not less than 0.001 µg and not more than 100 µg,
Method (I): dissolving the polymer A in the volatile solvent B to prepare a polymer solution having a concentration of 10% by mass; applying 0.005 g of the thus prepared polymer solution to a region of a surface of a polyethylene sheet having a width of 20 mm, a length of 100 mm and a thickness of 0.03 mm, the region having a width of 20 mm and a length of 50 mm which extends from an end of a short side of the polyethylene sheet along a length direction thereof and drying the polymer solution applied to the polyethylene sheet in a constant temperature oven set at 40 °C for 10 minutes to prepare the polyethylene sheet with a polymer film,
in which when a length of a portion of the polyethylene sheet to which the polymer solution is applied is represented by L1, and a length of a portion of the polyethylene sheet with the polymer film which undergoes deformation by drying the polymer solution applied thereto is represented by L2, a ratio of L2 to L1 [L2/L1] is defined as the deformation ratio of the polymer A.

2. The skin treatment method according to claim 1, wherein an amount of the fine liquid droplets applied in the step 1 per a unit area of the skin in terms of an amount of the polymer A applied is not less than 0.1 mg/cm² and not more than 3 mg/cm².

3. The skin treatment method according to claim 1 or 2, wherein a method of applying the fine liquid droplets to the skin in the step 1 is a method of ejecting the liquid composition by at least one method selected from the group consisting of an ink-jet method and a dispenser method to apply the fine liquid droplets to the skin.

4. The skin treatment method according to any one of claims 1 to 3, wherein an average diameter of the fine liquid droplets applied to the skin in the step 1 is not less than 10 µm and not more than 500 µm.

5. The skin treatment method according to any one of claims 1 to 4, wherein an interval between the fine liquid droplets applied to the skin in the step 1 is not less than 0.01 mm and not more than 2 mm.

6. The skin treatment method according to any one of claims 1 to 5, wherein a volatilization rate of the volatile solvent B as measured after volatilizing the volatile solvent B under a pressure of 1 atm at a temperature of 40 °C and a humidity of 60% RH for 30 minutes is not less than 5%.

7. The skin treatment method according to any one of claims 1 to 6, wherein the volatile solvent B is at least one solvent selected from the group consisting of water, an aliphatic alcohol comprising not less than 2 and not more than 4 carbon atoms, a volatile hydrocarbon oil and a volatile silicone oil.

8. The skin treatment method according to any one of claims 1 to 7, wherein a content of the polymer A in the liquid composition is not less than 0.1% by mass and not more than 30% by mass.

9. The skin treatment method according to any one of claims 1 to 8, wherein a mass ratio of the content of the polymer A to a content of the volatile solvent B (A/B) in the liquid composition is not less than 0.05 and not more than 0.3.

10. The skin treatment method according to any one of claims 1 to 9, wherein a total content of the polymer A and the volatile solvent B in the liquid composition is not less than 80% by mass and not more than 100% by mass.

11. The skin treatment method according to any one of claims 1 to 10, wherein the polymer A is at least one polymer selected from the group consisting of a silicone polymer comprising a structure represented by the following general formula (I), and a vinyl-based polymer comprising a repeating unit derived from an anionic group-containing vinyl-based monomer, 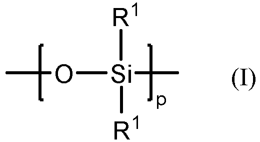 wherein R¹ groups are each independently a hydrocarbon group comprising not less than 1 and not more than 12 carbon atoms; and p is an integer of not less than 1.

12. A makeup method using the skin treatment method according to any one of claims 1 to 11 for improving wrinkles on skin.

13. The makeup method according to claim 12, wherein in the step 1, the fine liquid droplets of the liquid composition are applied to peripheral portions of the wrinkles on the skin.

14. A makeup method using the skin treatment method according to any one of claims 1 to 11 for forming undulation on skin.

15. A use of the skin treatment method according to any one of claims 1 to 11 for cosmetic surgical procedures.
